# EUROPÄISCHE PATENTSCHRIFT

(11) **EP 3 273 913 B1**
(45) Veröffentlichungstag und Bekanntmachung des Hinweises auf die Patenterteilung: **21.10.2020**
(21) Anmeldenummer: 16716171.0
(22) Anmeldetag: 23.03.2016
(51) Int. Cl.: A61F 2/38

(54) **OBERFLÄCHENERSATZIMPLANTAT DISTALER HUMERUS**
RESURFACING IMPLANT FOR THE DISTAL HUMERUS
IMPLANT DE RESURFAÇAGE D'HUMÉRUS DISTAL

(30) Priorität: 24.03.2015 DE 102015104442; 25.03.2015 DE 102015104480
(43) Veröffentlichungstag der Anmeldung: 31.01.2018
(73) Patentinhaber: OT Medizintechnik GmbH, 80639 München (DE)
(72) Erfinder: SCHREIBER, Ulrich, 80639 München (DE); LENICH, Andreas, 82152 Planegg (DE)
(74) Vertreter: Bobbert & Partner Patentanwälte PartmbB
(86) Internationale Anmeldenummer: PCT/EP2016/056450
(87) Internationale Veröffentlichungsnummer: WO 2016/151047

(56) Entgegenhaltungen:
- EP-A1- 0 098 466
- WO-A1-2011/035145
- DE-A1- 2 823 406
- US-A- 4 378 607

## Beschreibung

Die Erfindung bezieht sich auf eine medizinische Vorrichtung bzw. ein medizinisches Oberflächenersatzimplantat nach Anspruch 1, insbesondere ausgestaltet als Ellenbogengelenkersatz, partieller Ellenbogengelenkersatz, Ellenbogengelenkimplantat, totaler Gelenkersatz oder totaler Ellenbogengelenkersatz.

Gelenke unterliegen dem Verschleiß, der sowohl den Knorpel des Gelenks als auch gelenknahe Anteile des Knochens betreffen kann. Sind Knorpel und/oder Gelenk übermäßig geschädigt, dies kann auch aufgrund von Frakturen der Fall sein, so kann ein Gelenkersatz benötigt werden. Der geschädigte Gelenkknorpel und ggf. auch Teile des Knochens werden wo nötig entfernt, ein Gelenkersatz, zumeist aus Metall, wird in das Gelenk eingebracht und übernimmt, soweit möglich, die Funktion der geschädigten Strukturen, also von Knorpel und Knochen.

Aus der Offenlegungsschrift DE 28 23 406 ist ein künstliches Ellenbogengelenk bekannt.

Aufgabe der vorliegenden Erfindung ist es, einen Gelenkersatz oder ein Oberflächenersatzimplantat, insbesondere für das Ellenbogengelenk mit dem distalen Humerus und dem proximalen Radius, anzugeben.

Die erfindungsgemäße Aufgabe wird durch einen Ellenbogengelenkersatz mit den Merkmalen des Anspruchs 1 gelöst.

Erfindungsgemäß wird somit ein, insbesondere partieller, Ellenbogengelenkersatz vorgeschlagen.

Der Ellenbogengelenkersatz weist wenigstens ein, oder genau ein, Segment auf, insbesondere ein schalenförmiges, kalottenförmiges oder auf andere Weise gekrümmtes Segment, mit einer vollständig oder in wenigstens einem Abschnitt konkaven Innenkontur. Das Segment ist konfiguriert oder vorgesehen, um auf oder an wenigstens einer Knorpel- und/oder Knochenstruktur eines Gelenks eines Patienten angeordnet zu werden.

Das hierin rein bespielhaft als schalenförmig bezeichnete Segment weist einen lateralen Abschnitt und einen medialen Abschnitt auf (oder einen ersten Abschnitt und einen zweiten Abschnitt, wobei der erste Abschnitt nach Implantierung des Ellenbogengelenkersatzes lateral zum zweiten Abschnitt liegt, welcher wiederum medial zum ersten Abschnitt liegt). Beide Abschnitte sind entlang einer Längsrichtung des schalenförmigen Segments zueinander benachbart (mit oder ohne Kontakt zueinander oder in mittelbarem oder unmittelbarem Übergang) angeordnet.

Eine Außenkontur des Segments weist in Längsrichtung des Ellenbogengelenkersatzes oder des Segments wenigstens einen oder genau einen Wendepunkt auf.

Das hierin offenbarte Set ist nicht Teil der Erfindung. Es umfasst wenigstens einen erfindungsgemäßen Ellenbogengelenkersatz.

Es umfasst ferner wenigstens ein Werkzeug zum Bearbeiten von Knochenstrukturen. Das Werkzeug ist vorzugsweise ausgestaltet oder konfiguriert, um die Knochenstrukturen an die Innenkontur des Ellenbogengelenkersatzes anzupassen.

Das Werkzeug (oder: Instrument) kann ein Fräser, ein Schaber, ein Shaver oder ein anderes Werkzeug, insbesondere ein spanabhebendes Werkzeug sein.

Unter einem "Bearbeiten" von Knochenstrukturen kann ein Fräsen, Schaben, Abtragen, usw., Vorbereiten und/oder Anpassen verstanden werden.

Das hierin beschriebene Verfahren ist nicht Gegenstand der Erfindung. Es dient dem Bearbeiten von Knorpel- und/oder Knochenstrukturen zur Vorbereitung einer Implantation eines, insbesondere erfindungsgemäßen, partiellen Ellenbogengelenkersatzes.

Es umfasst das Einbringen eines Drahts, etwa eines Bohrdrahts, eines K-Drahts oder eines Kirschner-Drahts in den Knochen im Gelenkbereich.

Es umfasst weiter das Einbringen eines Werkzeugs zum Bearbeiten der Knochenstrukturen mithilfe des Drahts. Hierzu kann das Werkzeug über den Draht aufgefädelt werden.

Schließlich umfasst das Verfahren optional das Herausführen des Werkzeugs aus dem Gelenkbereich nach der Bearbeitung.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen sowie der Figuren.

Wenn hierin von einem Ellenbogengelenkersatz die Rede ist, so soll dies nicht beschränkend verstanden werden. In einigen erfindungsgemäßen Ausführungsformen ist das erfindungsgemäße Implantat zwar konfiguriert oder ausgestaltet, um als Ellenbogengelenkersatz verwendet zu werden. In anderen erfindungsgemäßen Ausführungsformen ist das erfindungsgemäße Implantat allerdings konfiguriert oder ausgestaltet, um als Gelenkersatz für andere Gelenke als das Ellenbogengelenk verwendet zu werden.

Das Anordnen des Segments auf oder an wenigstens einer Knochenstruktur des Patienten kann indirekt erfolgen (bei verbleibendem Restknorpel) oder direkt (ohne verbleibenden Knorpel).

In einigen erfindungsgemäßen, beispielhaften Ausführungsformen weisen die erfindungsgemäßen Gegenstände eines oder mehrere der hierin beschriebenen Merkmale in beliebiger Kombination auf, sofern eine solche Kombination für den Fachmann nicht erkennbar technisch unmöglich ist.

In einigen erfindungsgemäßen, beispielhaften Ausführungsformen wird unter einem Wendepunkt ein Punkt des Ellenbogengelenkersatzes verstanden (etwa auf seiner Außenfläche oder Außenkontur), an dem der Verlauf der Außenfläche oder Außenkontur (etwa bei Betrachtung des Ellenbogengelenksersatzs in einem Längsschnitt oder von der Seite) oder ein anderer dem Ellenbogengelenkersatz zuzuordnender Graph sein Krümmungsverhalten ändert: Der Verlauf oder der Graph wechselt hier entweder von einer Rechts- in eine Linkskurve oder umgekehrt.

In einigen erfindungsgemäßen, beispielhaften Ausführungsformen ist der Wendepunkt in Einklang mit seiner Bedeutung im Rahmen der mathematischen Kurvendiskussion zu verstehen.

In einigen erfindungsgemäßen, beispielhaften Ausführungsformen ist der Wendepunkt jener Punkt auf der Außenkontur oder dem Graphen, an welchem die Krümmung der Außenkontur oder des Graphen ihr Vorzeichen wechselt.

In einigen erfindungsgemäßen, beispielhaften Ausführungsformen ist der Wendepunkt jener Punkt, an welchem die Krümmung der Außenkontur oder des Graphen von konkav in konvex übergeht.

In einigen erfindungsgemäßen, beispielhaften Ausführungsformen liegt der Wendepunkt in einem Übergangsbereich zwischen dem lateralen Abschnitt und dem medialen Abschnitt.

Von einem partiellen Gelenkersatz kann erfindungsgemäß die Rede sein, wenn nur ein Teil oder eine Seite (lateral, medial, proximal, distal) eines am Gelenk beteiligten Knochens ersetzt wird. Ist beispielsweise nur ein Teil bzw. eine Seite des Ellenbogengelenks abgenutzt oder beschädigt, so mag es genügen, nur den abgenutzten oder beschädigten Teil des Gelenks zu erneuern. Geschieht dies, so ist hierin nur von einem partiellen Gelenkersatz bzw. einem einseitigen Oberflächenersatz die Rede. Nur ein Teil des Endes des am Gelenk beteiligten Knochens, etwa des Humerus, wird folglich ersetzt; der Gelenkersatz betrifft nicht die gesamte medial-lateral-Erstreckung des Knochens, etwa des Humerus. Der Gelenkersatz kann eine geringere Längsausdehnung als die Gelenkfläche haben. Die Längsausdehnung kann geringer sein als die Breite des Knochens.

Von einem totalen Gelenkersatz kann erfindungsgemäß die Rede sein, wenn der distale Humerus, insbesondere nur die komplette Oberfläche des Capitulum ohne Trochlea, und der proximale Radius bzw. Radiuskopf durch Implantate ersetzt werden.

Weiterhin kann von einem Gelenkersatz erfindungsgemäß die Rede sein, wenn zuvor implantierte Implantate des distalen Humerus und/oder des proximalen Radius durch andere oder weitere oder neue Implantate ersetzt werden.

In einigen erfindungsgemäßen, beispielhaften Ausführungsformen beziehen sich die Begriffe "lateral" und "medial" auf die Anordnung des Ellenbogengelenkersatzes im bestimmungsgemäß implantierten Zustand am Patienten. "Lateral" und "medial" werden dabei so verstanden, wie man diese Begriffe in Bezug auf den Patienten versteht.

In einigen erfindungsgemäßen, beispielhaften Ausführungsformen ist die Innenkontur des Segments die Innenseite, oder die konkave Seite, oder die dem Knochen zugewandte oder hiermit nach Implantation in indirektem Kontakt oder direktem Kontakt stehende Seite des Segments.

In einigen erfindungsgemäßen, beispielhaften Ausführungsformen weist die im Gebrauch des Gelenkersatzes zu der Gelenkfläche weisende Fläche eine polierte, abriebsfeste und damit tribologisch optimierte Oberfläche auf.

In manchen erfindungsgemäßen, beispielhaften Ausführungsformen weist die im Gebrauch des Gelenkersatzes zum Knochen zugewandte Innenseite Material- und/oder Oberflächeneigenschaften auf, die für den Fachmann als osteoinduktiv/-integrativ gelten. Die Osteoinduktivität kann durch eine Mikro- und/oder eine beispielsweise interkonnektierende Makrostruktur und/oder durch eine geeignete Materialwahl der Oberfläche erzielt oder gefördert werden.

In einigen erfindungsgemäßen, beispielhaften Ausführungsformen ist im Gebrauch des Gelenkersatzes zwischen dem aufbereiteten Knochenlager und dem Implantat eine feste Verbindung vorgesehen. Diese kann durch unterschiedliche Verankerungsprinzipien erzielt werden, z. B. durch Dorn, Kamm, Schraubenverbindung, winkelstabile Schraubenverbindung. Zusätzlich kann die Verbindung biologisch durch eine Interaktion zwischen Knochen und Implantat begünstigt werden, die sich nach und nach ausbilden und eine Langzeitstabilität begünstigen kann.

In einigen erfindungsgemäßen, beispielhaften Ausführungsformen weist der Gelenkersatz, insbesondere zum Knochenlager hin gewandt, resorbierbare Materialien auf, die nach Implantation des Gelenkersatzes degradieren, und welche die Stabilität begünstigen.

In einigen erfindungsgemäßen, beispielhaften Ausführungsformen ist der Gelenkersatz oder das Segment aus einer biokompatiblen Metall-Legierung gefertigt (CoCr-Legierung oder Ähnliches).

In einigen erfindungsgemäßen, beispielhaften Ausführungsformen ist das Material ein biokompatibles Metall, eine Keramik, ein Kunststoff oder eine Kombination aus diesen.

In einigen erfindungsgemäßen, beispielhaften Ausführungsformen ist das Metall z. B. eine CoCrMo-Legierung, und/oder eine Keramik z. B. ein Zirkoniumoxid oder ein Aluminiumoxid (Al₂O₃).

In einigen erfindungsgemäßen, beispielhaften Ausführungsformen ist der Kunststoff PEEK und/oder UHMWPE (ultrahochmolekulargewichtiges Polyethylen). Die Kunststoffe, insbesondere PEEK, können faserverstärkt sein, beispielsweise mittels Kohlenstofffasern und/oder Glasfasern.

In bestimmten erfindungsgemäßen, beispielhaften Ausführungsformen ist das Material ein kohlenstofffaserverstärkter Kunststoff (carbonfaserverstärkter Kunststoff, abgekürzt: CFK) oder weist ein solches Material auf.

In einigen erfindungsgemäßen, beispielhaften Ausführungsformen bilden der Ellenbogengelenkersatz auf Seiten des Humerus und ein Gelenkersatz auf Seiten des Radius eine Gleitpaarung.

In einigen erfindungsgemäßen, beispielhaften Ausführungsformen ist für den Gelenkersatz als Materialpaarung oder Gleitpaarung Metall-Kunststoff oder Keramik-Keramik vorgesehen. Dies trägt vorteilhaft zur Verringerung, Vorbeugung oder Vermeidung von Abrieb bei Gebrauch des Gelenkersatzes bei. Dies kann ferner zur Langlebigkeit des Ellenbogengelenkersatzes, zur Minderung der Infektionsgefahr oder Auftreten von allergischen Reaktionen beim Patienten beitragen.

In einigen erfindungsgemäßen, beispielhaften Ausführungsformen ist der Gelenkersatz oder ein Teil hiervon ausgestaltet, um als partieller oder - bevorzugt - totaler Ersatz des Radiuskopfes (Caput radii) zu dienen. Um die gewünschte Gelenkmechanik zu erhalten, basieren Größen und Proportionen vorzugsweise auf anatomischer Analyse.

In einigen erfindungsgemäßen, beispielhaften Ausführungsformen weist der Gelenkersatz eine Tellerform oder Scheibenform auf, beispielsweise bei Betrachtung des Gelenkersatzes von der Seite.

In einigen erfindungsgemäßen, beispielhaften Ausführungsformen weist der Gelenkersatz an seiner Oberfläche oder Außenkontur insbesondere auf einer oberen Fläche hiervon, wenigstens eine oder genau eine Vertiefung oder Tellertiefe auf.

Die Vertiefung oder Tellertiefe ist vorzugsweise an den Durchmesser und/oder die Höhe des Kopfes angepasst (variiert mit Implantatgrößen).

In einigen erfindungsgemäßen, beispielhaften Ausführungsformen ist die Außenwand des Kopfes des Gelenkersatzes gewölbt. Dies erlaubt vorteilhaft, dass der Gelenkersatz in oder an die Gelenkfläche der Ulna passt.

In einigen erfindungsgemäßen, beispielhaften Ausführungsformen weist der Gelenkersatz, am Knochen zugewandten Teil, eine Beschichtung, vorzugsweise zum Begünstigen einer Osteointegration, auf. Die Beschichtung kann mittels Reintitan-Plasma-Spray mit einer Schichtdicke von 40-50 µm und einer Rauheit von ca. 7 µm, aufgetragen sein. Die Beschichtung kann Hydroxylapatit (=Knochenzement) oder Ähnliches sein oder aufweisen. Die Beschichtung kann an allen erfindungsgemäßen Ausführungsformen erfolgen, beispielsweise am distalen Humerus (Capitulumimplantat) oder am proximalen Radius (Radiuskopfimplantat).

In einigen erfindungsgemäßen, beispielhaften Ausführungsformen weist der Gelenkersatz eine Verankerungsvorrichtung auf.

In einigen erfindungsgemäßen, beispielhaften Ausführungsformen weist die Verankerungsvorrichtung des Gelenkersatzes, insbesondere am proximalen Radius, wenigstens eine Spitze oder wenigstens einen Endabschnitt auf, welcher nach Implantation am weitesten distal zum Liegen kommt. Die Spitze oder der Endabschnitt können abgerundet sein, was einer unerwünschten Schädigung des Knochens (z. B. des Radius) bei der Implantation, und insbesondere beim Einführen, entgegenwirkt.

In manchen erfindungsgemäßen, beispielhaften Ausführungsformen weist die Verankerungsvorrichtung des Gelenkersatzes, insbesondere am distalen Humerus, wenigstens eine Spitze oder wenigstens einen Endabschnitt auf, welcher nach Implantation am weitesten proximal zum Liegen kommt. Die Spitze oder der Endabschnitt können abgerundet sein, was einer unerwünschten Schädigung des Knochens (z. B. des Humerus) bei der Implantation, und insbesondere beim Einführen, entgegenwirkt.

In einigen erfindungsgemäßen, beispielhaften Ausführungsformen weist die Verankerungsvorrichtung des Gelenkersatzes seitliche Einkerbungen zum Begünstigen der Osseointegration auf. Sie erlauben, dass der Knochen in sie hineinwächst, was die Implantation durch Ausbilden von Hinterschneidung stabiler macht.

In einigen erfindungsgemäßen, beispielhaften Ausführungsformen ist die Verankerungsvorrichtung als kegelförmiger Schaft, trapezförmiger Schaft, Keil, Widerhaken, Schraubenfixierung oder dergleichen ausgeführt.

In einigen erfindungsgemäßen, beispielhaften Ausführungsformen ist die Verankerungsvorrichtung als eine Vielzahl flexibler, elastischer oder biegsamer Spikes, vorzugsweise mit Rückstelleigenschaften ausgeführt. Dies erlaubt es den Spikes, sich beim Einbringen in den Markraum an die Geometrie oder die Oberfläche anzupassen. Sie werden beim Implantieren nach innen gebogen oder biegen sich nach innen. Indem sie aufgrund ihrer Flexibilität und der durch ihr Biegen auftretenden Spannung nach außen gegen den Knochen drücken, tragen sie zur Stabilität der Verankerung bei.

In einigen erfindungsgemäßen, beispielhaften Ausführungsformen weisen die Spikes oder die Spitze Widerhaken auf, die sich in den Knochen "eingraben", was zusätzlich vor einer unerwünschten Translation in axialer oder radialer Richtung, oder ganz allgemein vor einer Positionsveränderung, und vor einer unerwünschten Rotation schützen kann.

In einigen erfindungsgemäßen, beispielhaften Ausführungsformen weist der Gelenkersatz einen hier als kurzen Keil bezeichneten Keil auf. Obgleich der größte Beitrag zur Stabilität durch Aufliegen des Kopfes des Gelenkersatzes auf der Kortikalis des Radiushalses bewirkt wird, wird der kurze Keil zur Erhöhung der Stabilität in den Markraum oder - falls noch vorhanden - in den spongiösen Knochen eingebracht. Durch die geringe Höhe des Keils muss das Gelenk zur Implantation nur wenig aufgespreizt werden, was der Schonung der Weichteile/Bänder dienen kann und hilft, die natürliche Gelenkstabilität beizubehalten.

Der Keil kann Löcher oder Öffnungen haben, welche dem Einwachsen des Knochens und somit der langfristigen Stabilität des Implantats dienen können.

Der Keil kann im Querschnitt eine T-Form haben. Diese erlaubt hohe Stabilität des Bauteils an sich. Zudem kann sie ein filigranes Design ermöglichen.

Die laterale Seite des Keils kann nach innen abgerundet sein. Hierdurch kann das Implantat ohne - oder bei nur geringem - Aufspreizen des Gelenks und/oder Erweitern des Gelenkspalts eingebracht werden.

Die mediale Seite des Keils kann verglichen mit der lateralen Seite breiter sein. Sie kann abgerundet sein. Damit kann eine möglichst große Kontaktfläche zum Knochen erzielt werden.

Das Abrunden der Spitze des Keils kann vorteilhaft dazu beitragen, dass die Spitze bei ihrem Einführen den Knochen nicht ungewollt schneidet und der Knochen nicht zusätzlich geschädigt wird. Zum Kopf hin ist ein Plateau vorgesehen, so dass sich der Keil beidseitig am Knochen abstützen kann.

Der Keil kann eine Höhe von 10 bis 20 mm haben, vorzugsweise beträgt die Höhe etwa 15 mm (evtl. variabel bei verschiedenen Kopfgrößen).

In einigen erfindungsgemäßen, beispielhaften Ausführungsformen weist der Gelenkersatz Materialien auf, welche sogenannte Shape Memory Alloys (abgekürzt SMA), die auch als Formgedächtnislegierungen (abgekürzt FGL) bezeichnet werden können, oder Memorymetalle, wie beispielsweise Nitinol (abgekürzt NiTi), CobaltChrom, Verbundwerkstoffe, röntgentransparente Materalien und/oder Keramik umfassen.

In einigen erfindungsgemäßen, beispielhaften Ausführungsformen ist der Gelenkersatz spanend und/oder durch ein Gieß-, Umform- oder ein additives Verfahren gefertigt.

Die Implantatform kann zum Knochen hin (Implantatinnenkontur) zylindrisch (vorteilhaft weil man dadurch eine einfachere Knochenlagerbearbeitung hat und das Implantat aufgeschoben werden kann), dreieckig (siehe nachfolgende Ausführungen hierzu), zylindrisch mit einem nach medial oder nach lateral konischen Verlauf (vorteilhaft, da keine Translation in Richtung der Implantatlängsachse) sein, oder aber auch eine tonnenförmige oder eine der Gelenkfläche angepasste Form aufweisen (vorteilhaft durch Hinterschneidung).

In einigen erfindungsgemäßen, beispielhaften Ausführungsformen ist die Ausdehnung des lateralen Abschnitts (oder wenigstens eines Teilabschnitts oder aller Teilabschnitte hiervon) in Radialrichtung größer als die Ausdehnung des medialen Abschnitts (oder wenigstens eines Teilabschnitts oder aller Teilabschnitte hiervon) in Radialrichtung. Ebenso kann die Ausdehnung des lateralen Abschnitts (oder wenigstens eines Teilabschnitts oder aller Teilabschnitte hiervon) in Radialrichtung zunächst kleiner, dann aufsteigend bis zu einem Scheitelpunkt größer werdend und anschließend wieder abflachend kleiner werdend ausgeführt sein.

In einigen erfindungsgemäßen, beispielhaften Ausführungsformen weist das schalenförmige Segment in Umfangsrichtung des Segments keinen geschlossenen Umfang auf. Die Umfangsrichtung steht senkrecht auf der Längsrichtung und/oder senkrecht zur Radialrichtung des Segments.

In einigen erfindungsgemäßen, beispielhaften Ausführungsformen des Implantats des distalen Humerus beträgt die Dicke der Wand des schalenförmigen Segments zwischen 1 mm und 5 mm, insbesondere zwischen 2 mm und 3 mm. Dies trifft vorzugsweise auf jeden Wandabschnitt zu; vorzugsweise ist kein Wandabschnitt in solchen Ausführungsformen dicker als vorstehend angegeben.

In einigen erfindungsgemäßen, beispielhaften Ausführungsformen weist das Segment wenigstens in Abschnitten hiervon eine Außenkontur und/oder Innenkontur auf, welche in einer präoperativen Planungsstufe mittels Computerabbildung patientenspezifisch angepasst wurde. Die Außenkontur ist in einigen erfindungsgemäßen, beispielhaften Ausführungsformen vorzugsweise derart, dass sie zum angeborenen Verlauf eines entsprechenden Knochenteils eines Ellenbogengelenks eines Patienten identisch oder nachempfunden ist.

In einigen erfindungsgemäßen, beispielhaften Ausführungsformen weist das schalenförmige Segment eine raue Innenkontur-Oberfläche auf. Vorzugsweise beträgt die Oberflächenrauheit eine mittlere Rauheit Rₐ von ca. 7 µm. Derartige Rauheiten können, ebenso wie makroskopische Oberflächenbeschaffenheiten, wie beispielsweise interkonnektierende Oberflächenstrukturen, zur verbesserten Osteointegration beitragen oder führen.

In einigen erfindungsgemäßen, beispielhaften Ausführungsformen ist die Innenkontur des Segments in Längsrichtung analog der Außenkontur des Segments ausgebildet, entspricht dieser oder fährt dieser nach.

Erfindungsgemäß weist die Innenkontur des Segments in Längsrichtung wenigstens einen Wendepunkt auf.

In einigen erfindungsgemäßen, beispielhaften Ausführungsformen weist die Innenkontur des lateralen Abschnitts des Segments eine konkave, nicht-zylindrische Kontur auf. Die Innenkontur des medialen Abschnitts des Segments weist eine zylindrische Kontur in Längsrichtung auf.

In einigen erfindungsgemäßen, beispielhaften Ausführungsformen weist die Innenkontur des lateralen Abschnitts des Segments eine konkave, nicht-zylindrische Kontur auf. Die Innenkontur des medialen Abschnitts des Segments weist eine nach radial innen gewölbte, konvexe Kontur in Längsrichtung auf.

In einigen erfindungsgemäßen, beispielhaften Ausführungsformen weist die Innenkontur des lateralen Abschnitts des Segments eine konkave, nicht-zylindrische Kontur auf. Die Innenkontur des medialen Abschnitts des Segments weist eine nach radial innen gewölbte Kontur in Längsrichtung auf.

In einigen erfindungsgemäßen, beispielhaften Ausführungsformen weisen die Innenkontur des lateralen Abschnitts des Segments eine zylindrische Kontur und die Innenkontur des medialen Abschnitts des Segments eine konvexe Kontur in Längsrichtung oder eine konvexe Kontur in Umfangsrichtung auf.

In einigen erfindungsgemäßen, beispielhaften Ausführungsformen weist das schalenförmige Segment an seinem lateralen, stirnseitigen Endabschnitt eine Fixiereinrichtung auf, konfiguriert oder ausgestaltet, um das schalenförmige Segment mittels dieser Fixiereinrichtung am Knochen zu fixieren.

In einigen erfindungsgemäßen, beispielhaften Ausführungsformen weist die Fixiereinrichtung eine oder wenigstens eine Schraube auf oder besteht hieraus.

Das schalenförmige Segment kann eine Durchgangsöffnung aufweisen, vorgesehen zum Durchführen der Schraube oder eines Fadens zur Befestigung am Knochen.

Das schalenförmige Segment kann eine Durchgangsöffnung aufweisen, vorgesehen zum Durchführen eines Fadens zur Befestigung von Sehnen, Bändern oder anderen Weichteilstrukturen.

In einigen erfindungsgemäßen, beispielhaften Ausführungsformen weist die Fixiereinrichtung wenigstens je eine Lasche, einen Steg und/oder eine Strebe o. ä. als Schraubenhalterung auf oder besteht hieraus.

In einigen erfindungsgemäßen, beispielhaften Ausführungsformen ist die Fixiereinrichtung in Längsrichtung, parallel oder im Wesentlichen parallel zur Längsrichtung im Knochen fixierbar oder hierzu vorgesehen.

In einigen erfindungsgemäßen, beispielhaften Ausführungsformen sind das Segment oder Abschnitte hiervon aus einem - vorzugsweise biokompatiblen - metallischen Werkstoff und/oder einem keramischen Werkstoff und/oder einem Verbundwerkstoff und/oder einem Polymerwerkstoff (z.B. PEEK (Polyetheretherketone)) hergestellt oder weisen einen solchen auf.

In einigen erfindungsgemäßen, beispielhaften Ausführungsformen weist das Segment an seiner Innenseite wenigstens ein Befestigungselement zum Verankern des Ellenbogengelenkersatzes in einer Knochenstruktur auf.

In einigen erfindungsgemäßen, beispielhaften Ausführungsformen ist die Innenseite die Innenfläche des Segments, insbesondere des Implantats des distalen Humerus.

In einigen erfindungsgemäßen, beispielhaften Ausführungsformen verläuft die Längsachse parallel zu einer Drehachse des Ellenbogengelenks, und ist im implantierten Zustand des Ellenbogengelenkersatzes eine Drehachse des Ellenbogengelenks.

In einigen erfindungsgemäßen, beispielhaften Ausführungsformen ist die Außenseite eines weiteren Segments derart geformt, dass sie einer Fläche einer proximalen Ulna des Ellenbogengelenks konform oder kongruent ist.

In einigen erfindungsgemäßen, beispielhaften Ausführungsformen endet der Ellenbogengelenkersatz lateral mit einer Eintritts- oder Öffnungsebene oder Wandung, welche in einer Ebene liegt, die unter einem Winkel zur Längsachse des Ellenbogengelenkersatzes steht.

In einigen erfindungsgemäßen, beispielhaften Ausführungsformen liegt der Winkel in einem Bereich von 25° bis 45°, bevorzugt zwischen 30° und 40°, und beträgt insbesondere bevorzugt 35°, vorzugsweise genau oder etwa 35°.

In einigen erfindungsgemäßen, beispielhaften Ausführungsformen umfasst der Ellenbogengelenkersatz weiterhin eine Radiusprothese oder partielle Radiusprothese, insbesondere für den Radiuskopf (als Radiuskopfimplantat) oder Teile hiervon. Diese kann eine Verankerungsvorrichtung aufweisen und/oder eine Beschichtung aufweisen.

Das Verfahren umfasst optional das Einbringen des partiellen Ellenbogengelenkersatzes mithilfe eines Drahts, eines K-Drahts (der K-Draht kann als Kirschner-Draht bezeichnet werden) oder eines Bohrdrahtes, hierin auch kurz als Draht bezeichnet.

Der Draht wird vorzugsweise von lateral in die Rotationsachse oder in einen Rotationsabschnitt des Implantats des distalen Humerus eingebracht, vorzugsweise so dicht und so parallel an die Rotationsachse geführt wie möglich.

Ein Werkzeug, etwa ein Fräser oder ein Shaver wird über den Draht geschoben und über die Fläche des Knochens vorgeschoben, die es mit dem Werkzeug zu bearbeiten gilt.

Das Werkzeug hat vorzugsweise einen Gewebeschutz, welcher im Gebrauch zwischen Weichteilgewebe oder Gewebe, welches nicht mit dem Werkzeug behandelt werden soll, und dem zu bearbeitenden Knochen liegt.

Das Werkzeug hat ferner vorzugsweise einen rotierenden Werkzeugabschnitt, dessen Außenkontur entweder kugelig oder wiederum vorzugsweise kongruent zu der mittels des Werkzeugs zu erzielenden Außenkontur des Knochens ist. Die Rotationsachse des Werkzeugs liegt im Gebrauch vorzugsweise parallel zur Rotationsachse des Gelenks.

Das Werkzeug oder ein Halter für das Werkzeug kann eine Durchgangsöffnung zum Hindurchführen des vorstehend genannten Drahts haben. Das Werkzeug kann dann kontrolliert um die Rotationsachse bewegt werden. Durch diese Bewegung wird der Knochen abgetragen.

Das vorgestehend genannte Werkzeug kann allein verwendet werden. Von der Erfindung umfasst sind allerdings auch zwei- oder mehrstufige Vorgehensweisen, bei welchen z. B. zunächst ein zylindrischer Fräser eingebracht wird, z. B. über den Draht wie vorstehend beschrieben. Anschließend kann der individuelle, jedenfalls nicht-zylindrische Fräser eingebracht werden, der dem Knochen die gewünschte nicht-zylindrische Außenkontur gibt. Ein solches, vielstufiges Verfahren erfordert eine vergleichsweise geringe Arbeitshöhe für das Werkzeug im Gelenkbereich. Der Gelenkspalt muss weniger erweitert werden als beim oben beschriebenen Vorgehen, bei welchem genau ein Werkzeug zum Einsatz kommt. Dies kann eine Schonung der Weichteile bedeuten.

Das Verfahren umfasst optional das Herausführen des Bohrdrahtes und optional das Einbringen eines Befestigungselementes zum Verankern des Ellenbogengelenkersatzes in der Knochenstruktur.

In einigen erfindungsgemäßen, beispielhaften Ausführungsformen weist der Ellenbogengelenkersatz eine Längsrichtung, eine Querrichtung und eine Umfangsrichtung auf.

In einigen erfindungsgemäßen, beispielhaften Ausführungsformen weist der Ellenbogengelenkersatz zwei in Längsrichtung des Ellenbogengelenkersatzes einander gegenüberliegend abschließende mediale bzw. laterale Enden oder mediale oder laterale Endbereiche auf. Er weist ferner wenigstens oder genau einen gekrümmten Abschnitt, welcher in Umfangsrichtung des Ellenbogengelenkersatzes gekrümmt ist, auf. Alternativ besteht der den Ellenbogengelenkersatz aus dem in Umfangsrichtung des Ellenbogengelenkersatzes gekrümmten Abschnitt. Der gekrümmte Abschnitt kann das o. g. Segment des Ellenbogengelenkersatzes sein.

In einigen erfindungsgemäßen, beispielhaften Ausführungsformen ist der gekrümmte Abschnitt ausschließlich gekrümmt, z. B. ausschließlich konvex oder ausschließlich konkav gekrümmt.

In einigen erfindungsgemäßen, beispielhaften Ausführungsformen weist der Ellenbogengelenkersatz in einem in Längsrichtung des Ellenbogengelenkersatzes mittleren Bereich die geringste Krümmung auf.

In einigen erfindungsgemäßen, beispielhaften Ausführungsformen weist der gekrümmte Abschnitt in einem in Längsrichtung des Ellenbogengelenkersatzes mittleren Bereich seine größte Dicke auf.

In einigen erfindungsgemäßen, beispielhaften Ausführungsformen weist der Ellenbogengelenkersatz einen Kragenabschnitt auf, welcher medial zum gekrümmten Abschnitt liegt.

In einigen erfindungsgemäßen, beispielhaften Ausführungsformen sind die Innenseite und/oder die Außenseite des Kragenabschnitts medial breiter als lateral, wobei die Innenseite und/oder die Außenseite entlang der Längsachse von medial nach lateral abfällt.

In einigen erfindungsgemäßen, beispielhaften Ausführungsformen endet der Gelenkersatz medial und/oder lateral mit einer zur Längsachse senkrecht stehenden Eintritts- oder Öffnungsebene oder Wandung.

In einigen erfindungsgemäßen, beispielhaften Ausführungsformen weisen der Ellenbogenersatz oder der gekrümmte Abschnitt in der Längsrichtung eine Oberseite und eine Unterseite auf, wobei die Oberseite des Gelenkersatzes und/oder der gekrümmte Abschnitt länger als seine Unterseite ist.

In einigen erfindungsgemäßen, beispielhaften Ausführungsformen ist der gekrümmte Abschnitt an seiner Innenseite derart geformt, dass er einer Capitulum-Fläche eines distalen Humerus des Ellenbogengelenks konform ist und/oder einer Olecranon-fossa einer Rückfläche eines distalen Humerus eines Ellenbogengelenks konform oder zu dieser kongruent ist.

In manchen erfindungsgemäßen, beispielhaften Ausführungsformen ist der gekrümmte Abschnitt an seiner Innenseite derart geformt, dass er einer weiteren distalen Humerusgelenkfläche, insbesondere dem medialen Bereich jenseits des Capitulums des Humerus des Ellenbogengelenks konform ist oder zu dieser kongruent ist.

In bestimmten erfindungsgemäßen, beispielhaften Ausführungsformen ist der Ellenbogengelenkersatz ganz oder teilweise dazu vorbereitet oder ausgestaltet, mittels Knochenzement in den Knochenstrukturen fixiert zu werden. Dabei können die Oberflächen der Implantate, oder Abschnitte davon, für den distalen Humerus, insbesondere das Capitulumimplantat, und/oder für den proximalen Radius beschichtet oder nicht beschichtet, oberflächenbehandelt oder nicht oberflächenbehandelt, glatt oder nicht glatt sein. Insbesondere spezielle Verankerungsvorrichtungen der Implantate wie beispielsweise Schäfte unterschiedlicher Formen, Keile oder Haken können die beschriebenen Oberflächen aufweisen.

Manche oder alle erfindungsgemäßen Ausführungsformen können einen, mehrere oder alle der oben und/oder im Folgenden genannten Vorteile aufweisen.

Der Gelenkersatz kann sich durch die weitestgehende Kongruenz zu der nativen Form des Capitulums auszeichnen. Die unterschiedlichen Krümmungsradien der Gelenkfläche sind an dem Gelenkersatz vorteilhaft nachgeahmt (mindestens zwei unterschiedliche Radien). Aufgrund der Form der angrenzenden Gelenkfläche am Radius und der Form am distalen Humerus (Richtung Trochlea) ist es zudem als vorteilhaft anzusehen, wenn der Gelenkersatz nach medial mit einem "Kragenabschnitt" abschließt. Auf der lateralen Seite kann die Form durch die Verjüngung nach distal durch einen Winkel vorgegeben werden. Die Übertragung dieses Winkels auf die Implantatform kann einen positiven Effekt auf die daran vorbeiführenden Bänder haben. Durch die Form und die Abmessungen des Implantats kann eine erhöhte biomechanische Stabilität erreicht werden. Bänder werden bei der Implantation durch die Möglichkeit der weichteilschonenden und/oder minimal-invasiven Einbringung und durch die Lage und Abmessungen geschont.

Die vorliegende Erfindung wird im Folgenden anhand der beigefügten Figuren, in welcher identische Bezugszeichen gleiche oder ähnliche Bauteile bezeichnen, exemplarisch erläutert. In den jeweils schematisch vereinfachten Figuren gilt:
- **Fig. 1 a** - **e**: zeigen ein Capitulumimplantat eines erfindungsgemäßen partiellen Ellenbogengelenkersatzes in einer ersten beispielhaften Ausführungsform;
- **Fig. 2**: zeigt die Morphologie von Knochenstrukturen eines distalen Humerus-Bereichs;
- **Fig. 3 a - d**: zeigen ein Capitulumimplantat eines erfindungsgemäßen partiellen Ellenbogengelenkersatzes, der virtuell am Capitulum eines distalen Humerus-Bereichs implantiert ist;
- **Fig. 4 a - b**: zeigen ein Capitulumimplantat eines erfindungsgemäßen partiellen Ellenbogengelenkersatzes in einer zweiten beispielhaften Ausführungsform;
- **Fig. 5 a - c**: zeigen ein Capitulumimplantat eines erfindungsgemäßen partiellen Ellenbogengelenkersatzes in einer dritten beispielhaften Ausführungsform;
- **Fig. 6 a - c**: zeigen ein Capitulumimplantat eines erfindungsgemäßen partiellen Ellenbogengelenkersatzes in einer vierten beispielhaften Ausführungsform;
- **Fig. 7 a - c**: zeigen ein Capitulumimplantat eines erfindungsgemäßen partiellen Ellenbogengelenkersatzes in einer fünften beispielhaften Ausführungsform;
- **Fig. 8 a - b**: zeigen ein Capitulumimplantat eines erfindungsgemäßen partiellen Ellenbogengelenkersatzes in einer sechsten beispielhaften Ausführungsform;
- **Fig. 9 a - c**: zeigen ein Capitulumimplantat eines erfindungsgemäßen partiellen Ellenbogengelenkersatzes in einer siebten beispielhaften Ausführungsform;
- **Fig. 10 a - b**: zeigen ein Capitulumimplantat eines erfindungsgemäßen partiellen Ellenbogengelenkersatzes in einer achten beispielhaften Ausführungsform;
- **Fig. 11 a - b**: zeigen ein Capitulumimplantat eines erfindungsgemäßen partiellen Ellenbogengelenkersatzes mit einem kegelförmigen Schaft als Verankerungsvorrichtung;
- **Fig. 12 a - c**: zeigen ein Capitulumimplantat eines erfindungsgemäßen partiellen Ellenbogengelenkersatzes mit einem trapezförmigen Schaft als Verankerungsvorrichtung;
- **Fig. 13 a - c**: zeigen ein Capitulumimplantat eines erfindungsgemäßen partiellen Ellenbogengelenkersatzes mit einem Keil als Verankerungsvorrichtung;
- **Fig. 14 a - c**: zeigen ein Capitulumimplantat eines erfindungsgemäßen partiellen Ellenbogengelenkersatzes mit Widerhaken als Verankerungsvorrichtung;
- **Fig. 15 a - c**: zeigen ein Capitulumimplantat eines erfindungsgemäßen partiellen Ellenbogengelenkersatzes mit einem Steg zur Schraubenfixierung als Verankerungsvorrichtung;
- **Fig. 16 a - b**: zeigen ein Capitulumimplantat eines erfindungsgemäßen partiellen Ellenbogengelenkersatzes mit einem mittels eines Scharniers fixierten Steg zur Schraubenfixierung als Verankerungsvorrichtung;
- **Fig. 17 a - b**: zeigen ein Radiuskopfimplantat eines erfindungsgemäßen partiellen Ellenbogengelenkersatzes;
- **Fig. 18 a - c**: zeigen ein Radiuskopfimplantat mit einem keilförmigen Dorn als Verankerungsvorrichtung eines erfindungsgemäßen partiellen Ellenbogengelenkersatzes;
- **Fig. 19 a - b**: zeigen ein Radiuskopfimplantat mit einem Keil als Verankerungsvorrichtung eines erfindungsgemäßen partiellen Ellenbogengelenkersatzes;
- **Fig. 20 a - c**: zeigen ein Radiuskopfimplantat mit einem weiteren Keil als Verankerungsvorrichtung eines erfindungsgemäßen partiellen Ellenbogengelenkersatzes;
- **Fig. 21 a - c**: zeigen ein Radiuskopfimplantat mit einem weiteren Keil als Verankerungsvorrichtung eines erfindungsgemäßen partiellen Ellenbogengelenkersatzes;
- **Fig. 22 a - c**: zeigen schematisch die einzelnen Schritte zum Einbringen eines Radiuskopfimplantats in den proximalen Radius;
- **Fig. 23**: zeigt das Einführen eines K-Drahtes in den distalen Humerus zur Vorbereitung des Implantierens eines erfindungsgemäßen partiellen Ellenbogengelenkersatzes;
- **Fig. 24**: zeigt einen Fräskopf mit einem Gewebeschutz zum spanenden Abtragen von Knochenstrukturen;
- **Fig. 25**: zeigt den auf den K-Draht aufgeschobenen Fräskopf mit Gewebeschutz beim Abtrag von Knochenstrukturen; und
- **Fig. 26 a - f**: zeigen den erfindungsgemäßen partiellen Ellenbogengelenkersatz in verschiedenen Ansichten mit einem Capitulumimplantat als schalenförmigem Segment und einem Radiuskopfimplantat.

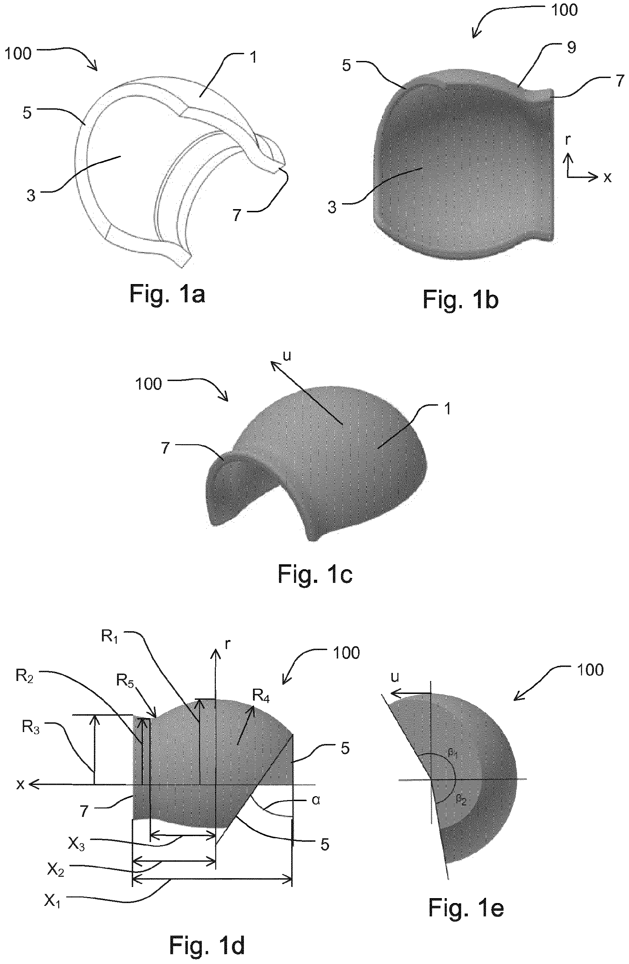

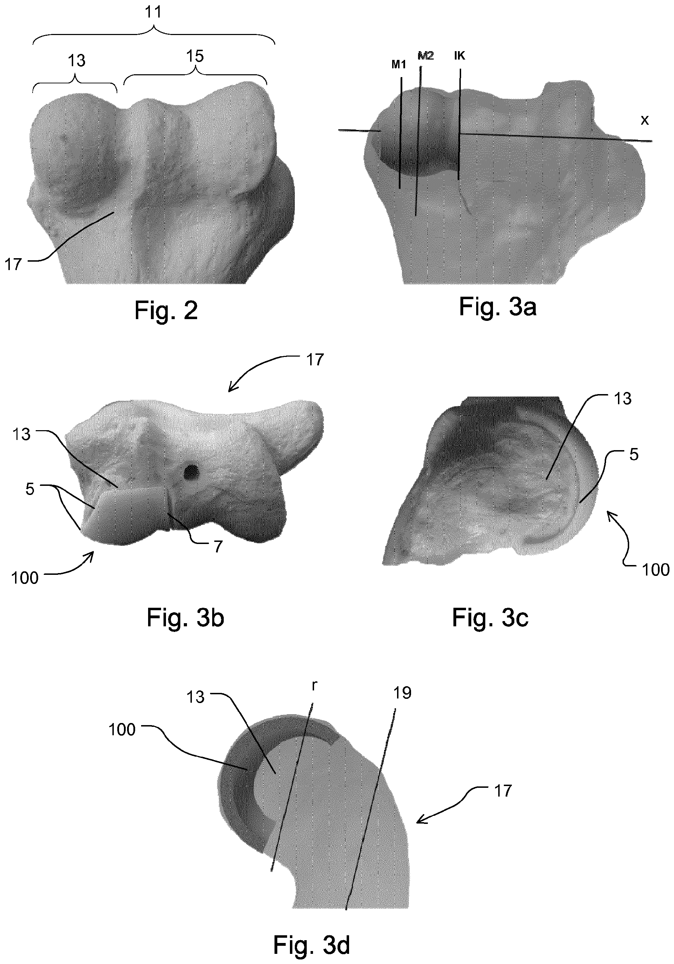

**Fig. 1a** zeigt ein Capitulumimplantat 100 eines erfindungsgemäßen partiellen Ellenbogengelenkersatzes 1000 für ein Ellenbogengelenk einer ersten beispielhaften Ausführungsform in einer perspektivischen Ansicht. Die genaue Anordnung des Capitulumimplantats 100 als Oberflächenersatz des Capitulum humeri am lateralen Abschnitt des distalen Humerus ist in den Figuren 3a bis 3d dargestellt und in Bezug zu diesen beschrieben.

Das Capitulumimplantat 100 wird im Folgenden vereinfachend als Implantat 100 bezeichnet.

Das Capitulumimplantat 100 kann als schalenförmiges Segment 100 bezeichnet werden.

Das Implantat 100 ist schalenförmig mit einer gewölbten äußeren Oberflächenkontur 1 (Außenkontur) und einer inneren Oberflächenform 3 (Innenkontur). Das Implantat 100 ist optional weder an einer lateralen Stirnseite 5 noch an einer medialen Stirnseite 7 hiervon geschlossen.

Die Dicke oder Stärke der Wand des Implantats 100, also der Abstand zwischen Außenfläche und Innenfläche des Implantats 100, ist in dieser ersten Ausführungsform über alle Abschnitte des Implantats 100 hinweg konstant.

In Umfangsrichtung u (s. Fig. 1c) ist die Schalenform optional ebenfalls nicht geschlossen, wie dies in den nachfolgenden Figuren näher beschrieben wird.

Die Längsrichtung des Implantats 100 ist mit x bezeichnet.

**Fig. 1b** zeigt das Implantat 100 der Fig. 1a in einer Seitenansicht, in der ein Wendepunkt 9 in der äußeren Oberflächenform in Längsrichtung x erkennbar ist. Der Wendepunkt 9 ist hier der Übergangspunkt zwischen der angenähert sphärischen Form im Bereich des Capitulum humeri (auf der lateralen Seite des Implantats) und dem sich medial anschließenden Bereich des Implantats 100 in Richtung zur Trochlea humeri (s. Fig. 2 und 3).

Die Innenkontur 3 des Implantats 100 ist annähernd anatomisch geformt, entsprechend der natürlichen Gelenkfläche, so dass vor einem Implantieren des Implantats 100 möglichst wenig Knochensubstanz entnommen oder abgefräst werden muss. Dies erlaubt eine verbesserte Durchblutung des Knochens verglichen mit Vorgehensweisen, bei denen mehr Knochensubstanz entfernt wird.

Ferner zeigt die Ansicht in Fig. 1b, dass die mediale Stirnseite 7 exemplarisch senkrecht zur Längsachse x abschließt, wogegen die laterale Stirnseite 5 mit einer Kurvenform abschließt, die in den nachfolgenden Figuren näher beschrieben wird.

**Fig. 1c** zeigt eine weitere perspektivische Ansicht des Implantats 100 der Fig. 1a von schräg oben auf die gewölbte äußere Oberflächenform 1 und die mediale Stirnseite 7.

**Fig. 1d** zeigt das Implantat 100 der Fig. 1a in einer Seitenansicht, in der das Implantat 100 gegenüber der Ansicht aus Fig. 1b um 180 Grad um eine Querachse (in Radialrichtung r) gekippt und um 90 Grad um ihre Langsachse x gedreht ist. Die mediale Stirnseite 7 ist links angeordnet, die laterale Stirnseite 5 rechts.

In dieser Ansicht ist die Form der lateralen Stirnseite 5 gut zu erkennen. Diese Form ist an den lateralen, anatomischen Abschluss des Capitulum humeri angepasst (siehe Fig. 2 und 3).

Zusammen mit der Ansicht in Fig. 1e werden einzelne exemplarische Maßangaben für die Dimensionierung unterschiedlicher Implantatgrößen angegeben. Diese exemplarischen Maßangaben resultieren aus der Vermessung mittels Computertomograhie-Aufnahmen (CT-Aufnahmen) von ca. 30 Patienten-Datensätzen distaler Humeri.

**Fig. 1e** zeigt eine um 90 Grad um die Radialachse r nach links gekippte und um die Längsachse x gedrehte Ansicht des Implantats 100 aus Fig. 1d.

Die mittels Patientendaten vermessenen Größen werden in die folgenden exemplarischen Implantatgrößen kategorisiert. Die Abkürzungen für die Implantatgrößen sind wie folgt:
S - Small; M - Medium; L - Large; XL - Extra-Large

| | S | M | L | XL |
|---|---|---|---|---|
| X₁ [mm] | 17,65 | 20,15 | 22,65 | 25,15 |
| X₂ [mm] | 10,18 | 11,51 | 12,85 | 14,19 |
| X₃ [mm] | 6,38 | 7,34 | 8,30 | 9,27 |
| R₁ [mm] | 8,60 | 9,80 | 11,00 | 12,20 |
| R₂ [mm] | 7,08 | 7,89 | 8,70 | 9,51 |
| R₃ [mm] | 7,63 | 8,58 | 9,52 | 10,47 |
| R₄ [mm] | 10,78 | 11,59 | 12,39 | 13,20 |
| R₅ [mm] | 3,93 | 4,06 | 4,18 | 4,31 |
| α [Grad; °] | 35 | 35 | 35 | 35 |
| β₁ [Grad; °] | 120 | 120 | 120 | 120 |
| β₂ [Grad; °] | 80 | 80 | 80 | 80 |

**Fig. 2** zeigt die Morphologie von Knochenstrukturen eines distalen Humerus-Bereichs. Gezeigt sind insbesondere das Ellenbogengelenk 11 (bzw. die Anteile des Humerus hieran) mit dem Capitulum 13 und der Trochlea 15, die den distalen Endbereich des Humerus 17 bilden. Beispielsweise kann bei einer Schädigung oder Verletzung des Ellenbogengelenks 11 im Bereich des Capitulum 13 die Oberfläche des Capitulum 13, etwa nach einem Abfräsen von Oberflächenschichten der Knochenstruktur, durch einen erfindungsgemäßen partiellen Ellenbogengelenkersatz 100 ersetzt werden, wie dies in den Fig. 3a bis 3d dargestellt ist.

**Fig. 3a** zeigt ein Capitulumimplantat 100 eines erfindungsgemäßen partiellen Ellenbogengelenkersatzes 1000, der virtuell am Capitulum 13 eines distalen Humerus-Bereichs implantiert ist. Die markierten Schnittebenen M1, M2 und IK sind beispielsweise Messebenen, die charakteristische Ebenen in bildgebenden Untersuchungen des Patienten bezeichnen. Die Messergebnisse können für den Chirurgen zum Implantieren eines erfindungsgemäßen partiellen Ellenbogengelenkersatzes 100 Hinweise für die zu wählende Größe des Implantats 100 geben (siehe Beschreibung zur Fig. 1, Implantatgrößen: S/ M/ L/ XL). Die Längsrichtung x kann als Rotationsachse bezeichnet werden. Die Rotationsachse kann sich auf das Implantat 100 sowie auf Trochlea und/oder Capitulum beziehen. Die markierten Schnittebenen M1, M2 und IK sind senkrecht (oder orthogonal) zur Rotationsachse angeordnet.

**Fig. 3b** zeigt einen Prototypen des Implantats 100, aufgesetzt auf ein Modell aus Kunststoff eines distalen Humerus, und ein Ellenbogengelenk. Das Implantat 100 ist auf das Capitulum 13 des distalen Humerus 17 aufgesetzt. In dieser Ansicht ist gut zu erkennen, wie sich der laterale Endbereich 5 und der mediale Endbereich 7 des Implantats 100 an die angrenzenden Knochenstrukturen anpassen bzw. sich an diese anfügen.

**Fig. 3c** zeigt die Ansicht aus Fig. 3b um 90 Grad nach rechts gedreht bzw. gekippt. Der Umfangswinkel um die Rotationsachse oder Längsachse x ist größer als 180 Grad, analog zur Fig. 1e, in der der Umfangswinkel 200 Grad beträgt (β₁: 120 Grad + β₂: 80 Grad), so dass das Implantat 100 zum Aufsetzen auf das Capitulum 13 leicht aufgeweitet werden muss, bevor es formschlüssig am Capitulum 13 anliegen kann.

**Fig. 3d** zeigt eine virtuelle Anordnung des Implantats 100 auf dem Capitulum 13. In dieser Ansicht sind die parallelen Achsen einer Längsachse 19 des Humerus 17, in der die Längsachse 19 insbesondere die Schaftachse 19 des Humerus 17 ist, und eine Achse in Radialrichtung r des Implantats 100, die gleichzeitig eine Radialrichtung r des Capitulum ist, gezeigt. Die Radialrichtung r steht senkrecht oder orthogonal zur Rotationsachse, oder Längsachse x, des Implantats 100.

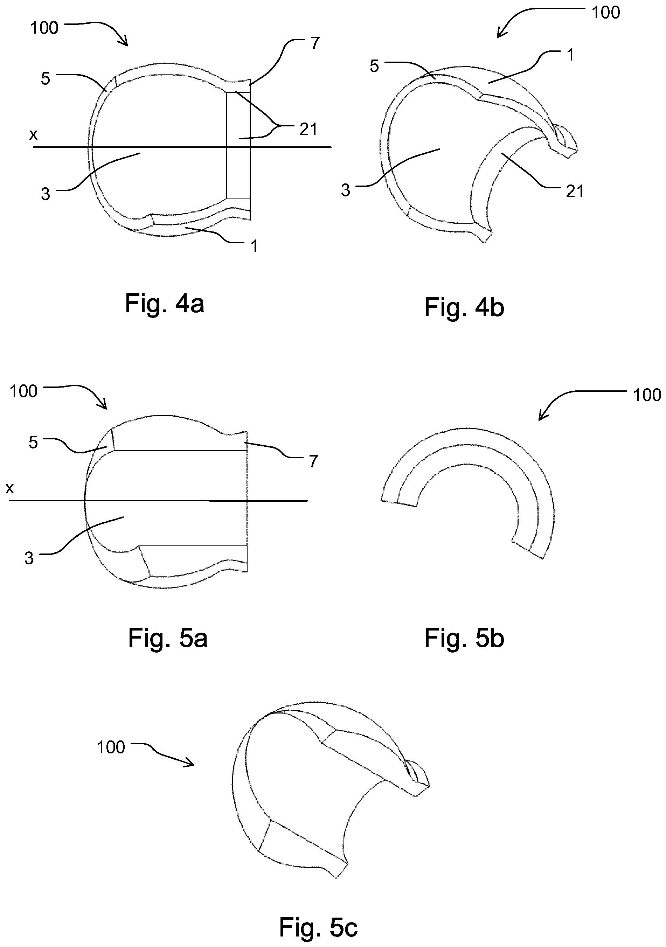

**Fig. 4a** zeigt ein Capitulumimplantat 100 eines erfindungsgemäßen partiellen Ellenbogengelenkersatzes 1000 in einer zweiten beispielhaften Ausführungsform. Gegenüber der Form und Kontur der ersten Ausführungsform des Implantats 100 aus Fig. 1a bis 1c weist die zweite Ausführungsform eine zylindrische Innenkontur 21 im medialen Endbereich 7 auf. Eine zylindrische Innenkontur 21 kann eine Bearbeitung der Knochenstruktur, beispielsweise mittels geeigneter spanabtragender Fräser, gegenüber einer durchgehend anatomisch geformten Kontur (siehe Fig. 1a bis 1c) vereinfachen. Eine anatomisch geformte Kontur kann aufgrund von konkaven Konturen und möglicherweise von Freiformflächen einen höheren Aufwand und möglicherweise eine längere Bearbeitung, auch der korrespondierenden Knochenstrukturen, bedeuten.

Eine Sicherung gegen eine translatorische Verschiebung des Implantats 100 entlang der x Richtung nach einer Positionierung auf dem Capitulum 13 kann durch die übrige Form der konkaven Innenkontur (lateralseitig) erreicht werden. Die Sicherung ist somit unabhängig von der Innenkonturform im medialen Endbereich 7. Allerdings kann eine anatomische Form (siehe Fig. 1a bis 1c) eine Verschiebung entlang der x-Richtung vorteilhaft zusätzlich blockieren und absichern.

Die Wandstärke des Implantats 100 im Bereich der zylindrischen Innenkontur 21 kann gegenüber einer anatomisch geformten Innenkontur - zumindest in Abschnitten der Innenkontur - größer sein und möglicherweise eine erhöhte Stabilität aufweisen.

Ein Aufschieben des Implantats 100 der zweiten Ausführungsform gemäß Fig. 4a und Fig. 4b von lateral (in Fig. 4a von links nach rechts) auf das Capitulum 13 kann aufgrund der zylindrischen Innenkontur 21 einfacher oder sogar erstmals möglich werden gegenüber der anatomischen Form nach Fig. 1a bis Fig. 1c. Die anatomische Form kann aufgrund der Hinterschneidung im medialen Endbereich 7 ein lateralseitiges Aufschieben verhindern oder zumindest erschweren.

Eine anatomisch geformte Innenkontur (siehe Fig. 1a bis Fig. 1c) kann das Maß einer notwendigen Knochenresektion (Abtragen von Knochenstrukturen zum Anpassen an die Implantatform) gegenüber der zylindrischen Innenkontur 21 verringern und minimieren.

**Fig. 4b** zeigt eine perspektivische Ansicht der zweiten Ausführungsform des Implantats 100.

**Fig. 5a** zeigt ein Capitulumimplantat 100 eines erfindungsgemäßen partiellen Ellenbogengelenkersatzes 1000 in einer dritten beispielhaften Ausführungsform. In dieser dritten Ausführungsform ist die Innenkontur 3 (oder innere Oberflächenform) durchgehend, also vom medialen 7 bis zum lateralen Endbereich 5, zylindrisch.

Eine zylindrische Innenkontur kann die Bearbeitung der Knochenstrukturen, auf die das Implantat 100 aufgesetzt wird, vereinfachen und erleichtern. Eine zylindrische Form ist in der Regel, beispielsweise mittels Fräsen, einfacher zu erzielen als eine anatomische Kontur, wie sie in Fig. 1a bis Fig. 1c dargestellt ist. Allerdings kann ein erhöhtes Abtragsvolumen von Knochenstrukturen aus anderen Gründen (beispielsweise eine erhöhte Knochenstabilität als Basis für die Stabilität des Implantats 100) unerwünscht sein. Daher können rein exemplarisch Mindest-Innenradien für die zylindrische Innenkontur angegeben werden, die hinsichtlich der übrigen Abmessungen Bezug auf die Tabelle zur Fig. 1e nehmen (siehe oben). Diese Mindest-Innenradien sollen eine ausreichende Knochensubstanz zum Fixieren und Stabilisieren des Implantats 100 gewährleisten. Die im Folgenden angegebenen Werte sind rein exemplaisch zu verstehen. Für die Implantatkategorie S (Small) kann ein Mindestinnenradius von 5,4 mm angegeben werden, für M (Medium) von 6,0 mm, für L (Large) von 6,6 mm und für XL (Extra-Large) von 7,2 mm. Damit liegt die Wandstärke des Implantats, insbesondere in belasteten Bereichen (durch natürliche Armbewegungen und daraus resultierenden Kräften auf das Elenbogengelenk), ca. zwischen 3,2 mm (für S) und 5 mm (für XL).

Eine zylindrische Innenkontur kann ein lateralseitiges Aufschieben des Implantats 100 auf das Capitulum 13, insbesondere bei einem Umfangswinkel ß von größer als 180 Grad (siehe Fig. 1e) vereinfachen oder sogar erst ermöglichen.

Eine zylindrische Innenkontur kann jedoch eine Sicherung und/oder Verankerung gegen translatorische Bewegungen entlang der Rotationsachse (Längsachse x) und eine Rotation des Implantats 100 um die Rotationsachse (in Umfangsrichtung u) nicht oder weniger zuverlässig sicherstellen, ohne dass zusätzliche Fixierungen, beispielsweise mittels Schrauben, oder Verankerungen des Implantats 100 erfolgen.

**Fig. 5b** zeigt eine Seitenansicht und **Fig. 5c** zeigt eine perspektivische Ansicht des dritten Ausführungsbeispiels des Implantats 100. In Fig. 5b wird verdeutlicht, dass der Umfangswinkel ß (siehe die Beschreibung zur Fig. 1e) größer als 180 Grad ist.

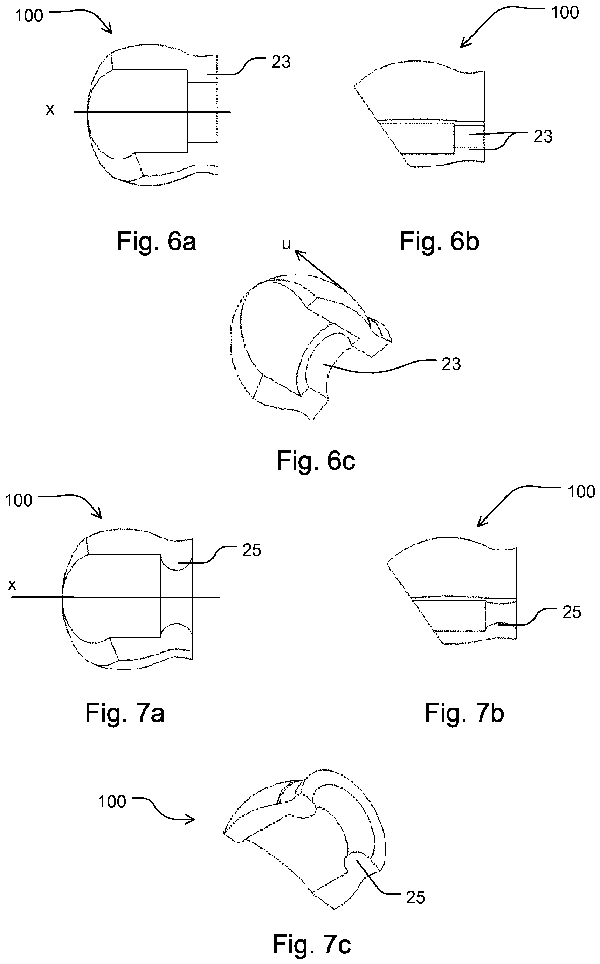

**Fig. 6a** zeigt ein Capitulumimplantat 100 eines erfindungsgemäßen partiellen Ellenbogengelenkersatzes 1000 in einer vierten beispielhaften Ausführungsform. Gegenüber der dritten Ausführungsform weist die vierte Ausführungsform einen nach innen versetzten, in Umfangsrichtung u angeordneten zylindrischen Absatz 23 auf. Der versetzte, zylindrische Absatz 23 kann als Abschnitt mit einem zylindrischen Versatz mit einem kleineren Radius gegenüber der weiter lateral angeordneten zylindrischen Form bezeichnet werden. Die Beschreibung und Diskussion zur Fig. 5a gelten weitgehend analog zur Fig. 6a. Allerdings kann das Implantat 100 der vierten Ausführungsform nicht von lateral in Längsrichtung x auf das Capitulum 13 aufgeschoben werden, sondern muss von vorne (anterior) aufgesetzt werden. Dafür ist es notwendig, dass der Umfangswinkel ß (siehe Fig. 1e) max. 180 Grad beträgt. Nach dem Aufsetzen auf das Capitulum 13 ist das Implantat 100 gegen ein Verschieben (translatorische Bewegung in x-Richtung) auf dem Capitulum 13 des Ellenbogengelenks gesichert.

**Fig. 6b** zeigt eine um ca. 90 Grad um die Längsachse x gedrehte Seitenansicht und **Fig. 6c** eine perspektivische Ansicht des vierten Ausführungsbeispiels des Implantats 100.

**Fig. 7a** zeigt ein Capitulumimplantat 100 eines erfindungsgemäßen partiellen Ellenbogengelenkersatzes 1000 in einer fünften beispielhaften Ausführungsform. Diese Ausführungsform ist der vierten Ausführungsform (Fig. 6) sehr ähnlich, weshalb die Beschreibung und Diskussion zur Fig. 6 analog auch hier gilt. Lediglich der nach innen versetzte zylindrische Absatz 23 aus Fig. 6a ist in Fig. 7a halbkreisförmig ausgeführt. Der nach innen angeordnete Versatz kann als halbkugelförmige Einkerbung 25 bezeichnet werden. Dies hat beispielsweise Auswirkungen auf die Form der Bearbeitung der Knochensubstanz in diesem Bereich und erfordert eine modifizierte Fertigung des Implantats 100.

Die Einkerbung 25 kann alternativ zu der Halbkugelform eine andere Form aufweisen.

**Fig. 7b** zeigt eine um ca. 90 Grad um die Längsachse x gedrehte Seitenansicht und **Fig. 7c** eine perspektivische Ansicht des fünften Ausführungsbeispiels des Implantats 100.

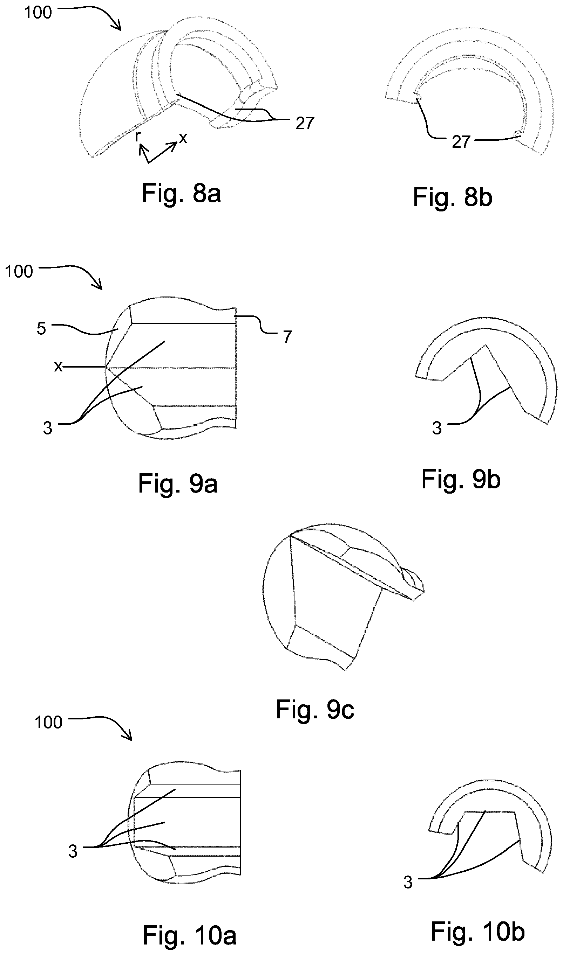

**Fig. 8a** zeigt ein Capitulumimplantat 100 eines erfindungsgemäßen partiellen Ellenbogengelenkersatzes 1000 in einer sechsten beispielhaften Ausführungsform in einer perspektivischen Ansicht. Gegenüber der vierten (Fig. 6) und fünften (Fig. 7) Ausführungsform weist die sechste Ausführungsform zwei auf dem Umfang gegenüber angeordnete, nach innen versetzte, halbkugelförmige Einkerbungen 27 in Längsrichtung x auf. Die Einkerbungen 27 sind jeweils an den beiden Endbereichen des in Umfangsrichtung u nicht geschlossenen Implantats 100 angeordnet. Insbesondere in Fig. 8b wird deutlich, dass der Umfangswinkel ß größer als 180 Grad ist, so dass das Implantat entweder lateralseitig auf das Capitulum 13 aufgeschoben werden kann oder radialseitig durch ein (elastisches) Aufspreizen des Implantats 100 aufgesetzt wird.

Die Einkerbung 27 kann alternativ zu der Halbkreisform eine andere Form aufweisen.

**Fig. 8b** zeigt das Implantat 100 stirnseitig von medial.

**Fig. 9a** zeigt ein Capitulumimplantat 100 eines erfindungsgemäßen partiellen Ellenbogengelenkersatzes 1000 in einer siebten beispielhaften Ausführungsform. Gegenüber der dritten Ausführungsform (Fig. 5) ist die Innenkontur (oder innere Oberflächenform 3) durchgehend, vom medialen Endbereich 7 bis zum lateralen Endbereich 5, flächenförmig ausgestaltet. Diese Flächenform kann ein Verdrehen des Implantats 100 nach seinem Aufsetzen auf das Capitulum 13 verhindern. Das Implantat 100 ist gegen eine Rotation um die Längsachse x gesichert. Vor einer Implantation kann die Knochenstruktur des Capitulum 13 von lateral beispielsweise mit Hilfe einer Sägeschablone bearbeitet und zugesägt werden. Anschließend kann das Implantat 100 von lateral aufgeschoben werden.

**Fig. 9b** zeigt das Implantat 100 stirnseitig von lateral, und **Fig. 9c** in einer perspektivischen Ansicht.

**Fig. 10a** zeigt ein Capitulumimplantat 100 eines erfindungsgemäßen partiellen Ellenbogengelenkersatzes 1000 in einer achten beispielhaften Ausführungsform. Gegenüber der Ausführungsform der Fig. 9a weist die Innenkontur 3 zusätzliche Flächen auf und ist im Querschnitt oder in einem Schnitt senkrecht zur Längsachse x trapezförmig ausgestaltet. Die Innenkontur 3 kann zwei, drei oder mehr Flächen aufweisen.

**Fig. 10b** zeigt das Implantat 100 stirnseitig von medial.

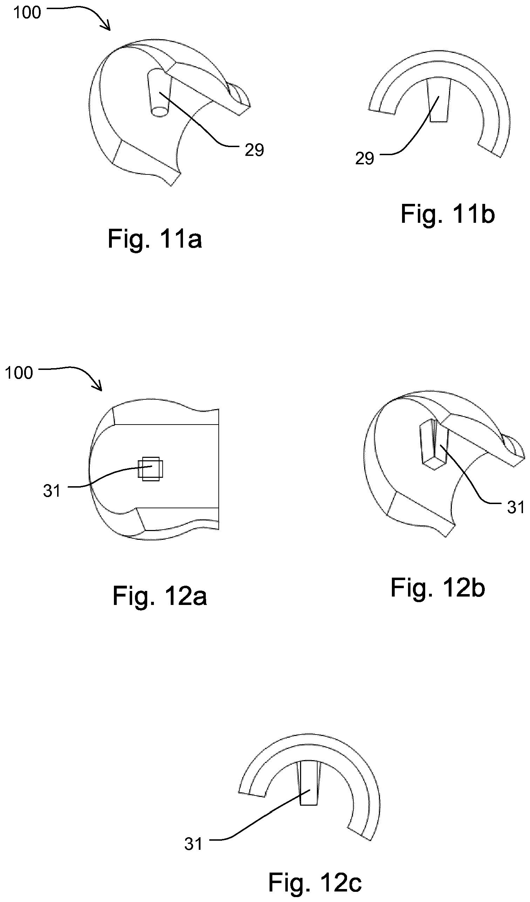

**Fig. 11a** zeigt ein Capitulumimplantat 100 eines erfindungsgemäßen partiellen Ellenbogengelenkersatzes 1000 mit einem kegelförmigen Schaft 29 als Verankerungsvorrichtung.

Die in den Figuren 11 bis 16 dargestellten Verankerungsmechanismen können mit den gezeigten und beschriebenen Ausführungsformen der Innenkonturen (Fig. 1, 4, 5, 6, 7, 8, 9, 10) kombiniert werden. Die Verankerungsmechanismen können im Einzelnen jeweils an Ecken und Kanten optional abgerundet werden und/oder die Oberfläche (des jeweiligen Verankerungsmechanismus) durch deren Oberflächenrauheit und/oder eine optionale Beschichtung für die Osteointegration optimiert werden.

Alle in den Figuren 11 bis 16 dargestellten Verankerungsmechanismen können eine translatorische Bewegung in x-Richtung und/oder eine rotatorische Bewegung um die Längsachse x des Implantats 100 verhindern.

Die Knochenstrukturen können für die in den Figuren 11 bis 16 dargestellten Verankerungsmechanismen beispielsweise mittels einer Reibahle, einer Raspel und/oder einer Säge oder Ähnlichem präpariert werden. Der jeweilige Verankerungsmechanismus kann mittels sogenanntem Press-fit in das Capitulum oder allgemein in die Knochensubstanz eingepresst oder eingeschlagen werden. Dies kann die Osteointegration des Implantats 100 verbessern oder beschleunigen.

Bei den Verankerungsmechanismen können Hinterschneidungen vorgesehen werden, in die die Knochenstrukturen einwachsen können und so eine langfristige Stabilität des Implantats 100 erhöhen.

Die Position des jeweiligen Verankerungsmechanismus ist insbesondere am maximalen Radius des Capitulums und/oder am größten Radius des Implantats angeordnet und vorgesehen. Die genaue Position und/oder der Winkel, in dem der jeweilige Verankerungsmechanismus in die Knochenstruktur eingebracht wird, können je nach intraoperativer Situation am Patienten variieren.

**Fig. 11b** zeigt das Implantat 100 mit dem kegelförmigen Schaft 29 stirnseitig von lateral.

In den **Fig. 12a, Fig. 12b** und **Fig. 12c** werden ein Capitulumimplantat 100 eines erfindungsgemäßen partiellen Ellenbogengelenkersatzes 1000 mit einem trapezförmigen Schaft 31 als Verankerungsvorrichtung dargestellt.

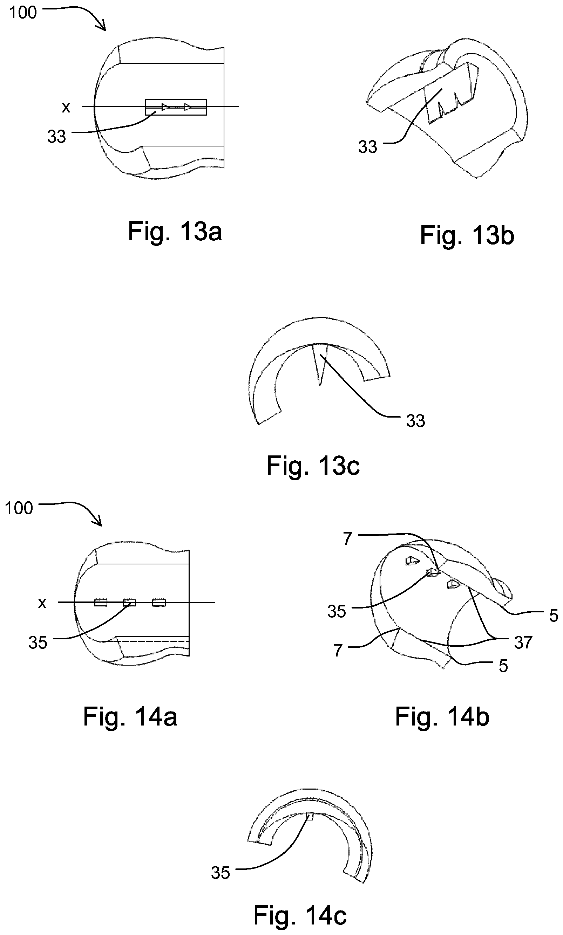

In den **Fig. 13a, Fig. 13b** und **Fig. 13c** werden ein Capitulumimplantat 100 eines erfindungsgemäßen partiellen Ellenbogengelenkersatzes 1000 mit einem Keil 33 als Verankerungsvorrichtung dargestellt.

Der Keil 33 verläuft insbesondere parallel zur Rotationsachse x des Implantats 100 und des Ellenbogengelenks. Der Keil 33 kann ein sogenanntes Sägezahn-Profil aufweisen, welches ein Verschieben des Implantats 100 nach lateral verhindern kann.

Zum Einsetzen des Implantats 100 mit dem Keil 33 als Verankerungsvorrichtung in den Knochen kann zur Knochenvorbereitung eine Kerbe in den Knochen eingesägt werden. Das Implantat 100 kann dadurch von lateral aufgeschoben werden, ohne dass der Knochen des Ellenbogengelenks aufgespreizt werden muss.

In den **Fig. 14a, Fig. 14b** und **Fig. 14c** werden ein Capitulumimplantat 100 eines erfindungsgemäßen partiellen Ellenbogengelenkersatzes 1000 mit Widerhaken 35 als Verankerungsvorrichtung dargestellt.

Die Widerhaken 35 sind an der Innenseite des Implantats 100 angeordnet. Die Widerhaken 35 können, wie in Fig. 14 dargestellt, kantig oder alternativ kegelförmig ausgestaltet sein. Die radiale Ausdehnung oder Höhe kann exemplarisch auf 3 mm begrenzt sein.

In den Knochen kann das Implantat 100 vorteilhaft ohne weitere Vorbehandlung eingesetzt werden. Eine Vorbohrung oder ähnliches ist aufgrund der kleinen Ausmaße der Widerhaken 35 nicht notwendig.

Die Widerhaken 35 können eine Primärstabilität des Implantats 100 gegen eine Translation (in Längsrichtung x) und/oder gegen eine Rotation (um die Längsachse x) bewirken.

Die Widerhaken 35 können alternativ oder ergänzend seitlich an den Kanten in Umfangsrichtung 37 und/oder nur lateral oder medial angeordnet sein.

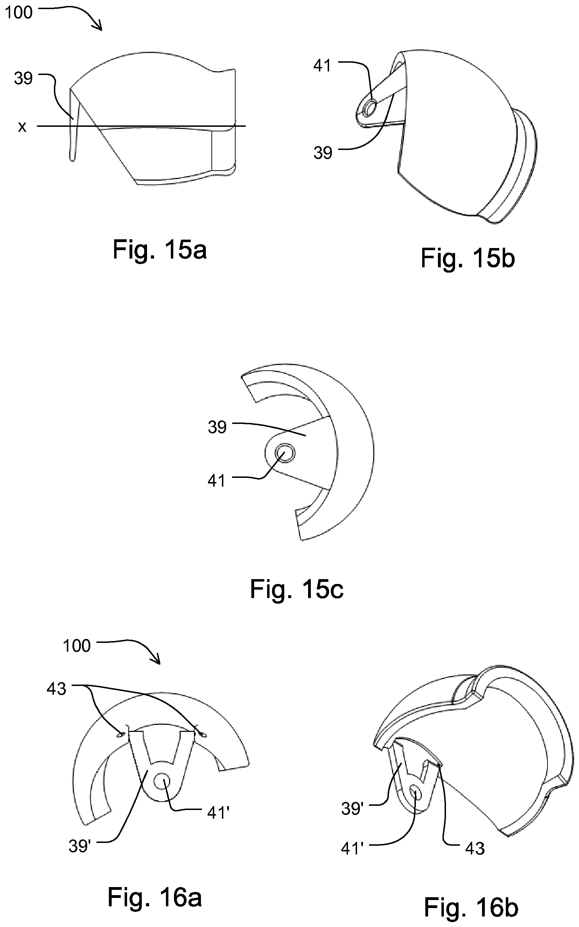

In den **Fig. 15a, Fig. 15b** und **Fig. 15c** werden ein Capitulumimplantat 100 eines erfindungsgemäßen partiellen Ellenbogengelenkersatzes 1000 mit einem Steg 39 zur Schraubenfixierung als Verankerungsvorrichtung dargestellt. Der Steg 39 kann als Lasche bezeichnet werden.

Der Steg 39 ist stirnseitig lateral an dem Implantat 100 angeordnet, insbesondere außerhalb der eigentlichen Gelenkfläche. Der Steg 39 weist eine Öffnung 41 oder Bohrung auf, die ca. in Höhe der Rotationsachse x angeordnet ist. Durch die Öffnung 41 kann eine Schraube in den Knochen eingebracht werden, die das Implantat 100 in seiner Position hält und fixiert. Der Schraubendurchmesser kann rein exemplarisch ca. 2 mm betragen. Bei einer Fixierung oder Verankerung des Implantats 100 mittels des Stegs 39 und einer durch die Öffnung 41 eingeführten Schraube ist insbesondere keine weitere Vorbereitung, beispielsweise in Form einer Vorbohrung des Knochens, notwendig.

Zur Implantation kann das Implantat 100 beispielsweise mit Hilfe der Öffnung 41 über einen Führungsdraht, der auch zur Bearbeitung (mittels Fräsen und Sägen) der Knochenstrukturen des Capitulums und/oder des Ellenbogengelenks einsetzbar ist, geführt und auf das Capitulum geschoben werden. Anschließend kann der Führungsdraht entnommen und die Schraube zur Fixierung des Implantats eingebracht werden.

In den **Fig. 16a** und **Fig. 16b** werden ein Capitulumimplantat 100 eines erfindungsgemäßen partiellen Ellenbogengelenkersatzes 1000 mit einem mittels eines Scharniers 43 fixierten Steg 39' zur Schraubenfixierung als Verankerungsvorrichtung dargestellt.

Der Steg 39' ist mittels des Scharniers 43 am Implantat 100 befestigt. Aufgrund der durch das Scharnier 43 gegebenen Drehmöglichkeit des Stegs 39' relativ zu dem Implantat 100 kann die Anordnung Implantat 100 und Steg 39' an die anatomischen Verhältnisse des Capitulums vorteilhaft angepasst und optimiert werden. Weiterhin kann das Implantat 100 optional mit wenigstens einer polyaxialen Schraube zusätzlich fixiert werden.

Optional kann der Steg 39' lediglich zur geführten Applikation des Implantats 100 an die Implantationsposition am Capitulum verwendet werden, um den Steg 39' nach der Positionierung von dem Implantat 100 zu entkoppeln und zu entfernen. Beispielsweise könnte, je nach Situation während einer Implantation, ein anderer Verankerungsmechanismus gewählt werden oder aufgrund der Innenkontur kein weiterer Verankerungsmechanismus notwendig sein.

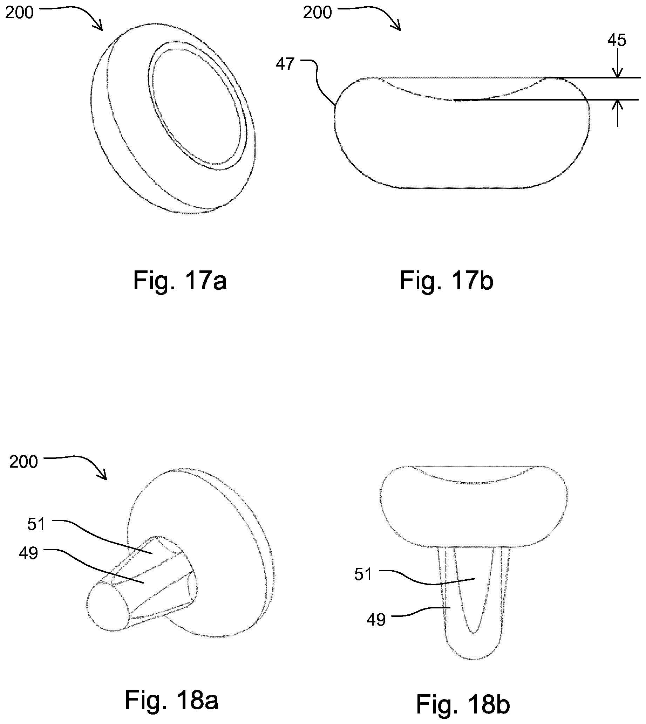

**Fig. 17a** zeigt ein Radiuskopfimplantat 200 eines erfindungsgemäßen partiellen Ellenbogengelenkersatzes 1000.

Das Radiuskopfimplantat 200 ist zum Ersatz des Radiuskopfes (proximal, ellbogennah) vorgesehen. Ziel einer Implantation eines Radiuskopfimplantats 200 ist es, die Gelenkmechanik, beispielsweise infolge einer Arthrose oder nach einem traumatischen Ereignis wie beispielsweise einem Unfall, zumindest teilweise wiederherzustellen. Insbesondere basieren die Größen und Proportionen des erfindungsgemäßen Radiuskopfimplantats 200 auf einer anatomischer Analyse.

Das Radiuskopfimplantat 200 weist eine Tellertiefe 45 auf, die an den Radius (siehe Tabelle zu Fig. 1e, z. B. R₄) und die Höhe des Capitulumimplantats 100 angepasst wird (variiert mit Implantatgrößen S/ M/ L/ XL; siehe Tabelle zu Fig. 1e). Die Außenwand 47 des Radiuskopfimplantats 200 ist gewölbt, so dass das Radiuskopfimplantat 200 in die Gelenkfläche zur Ulna (der Unterarmknochen umfasst die sogenannte Speiche oder Radius und die sogenannte Elle oder Ulna, die zusammen mit dem Capitulum und der Trochlea im Wesentlichen das Ellenbogengelenk ausbilden) passt.

Das Radiuskopfimplantat 200 kann aus einem oder mehreren biokompatiblen Materialien hergestellt sein, wie beispielsweise aus Metall, Keramik, Kunststoff, einem Verbundwerkstoff und/oder einer Kombination aus diesen Materialien. Beispiele für biokompatible Materialien sind die Metalllegierung Kobalt-Chrom-Molybdän, die Keramiken Zirkoniumoxid, Aluminiumoxid (Al₂O₃) oder die Kunststoffe Polyetheretherketon (PEEK), Ultrahochmolekulargewichtiges Polyethylen (UHMWPE). Rein exemplarische Gleitpaarungen mit dem Capitulumimplantat 100 und dem Radiuskopfimplantat 200 könnte eine Metall-Kunststoff oder Keramik-Keramik Paarung sein. Diese Gleitpaarungen sind als abriebarme Materialpaarungen bekannt und können daher vorteilhaft als dauerhafte Materialpaarung verwendet werden. Weiterhin können derartige Materialpaarungen eine geringe Infektionsgefahr und/oder das Vermeiden von allergischen Reaktionen belegen.

**Fig. 17b** zeigt eine Seitenansicht des Radiuskopfimplantats 200.

**Fig. 18** **a** - **b** zeigen ein Radiuskopfimplantat 200 mit einem keilförmigen Schaft 49 als Verankerungsvorrichtung.

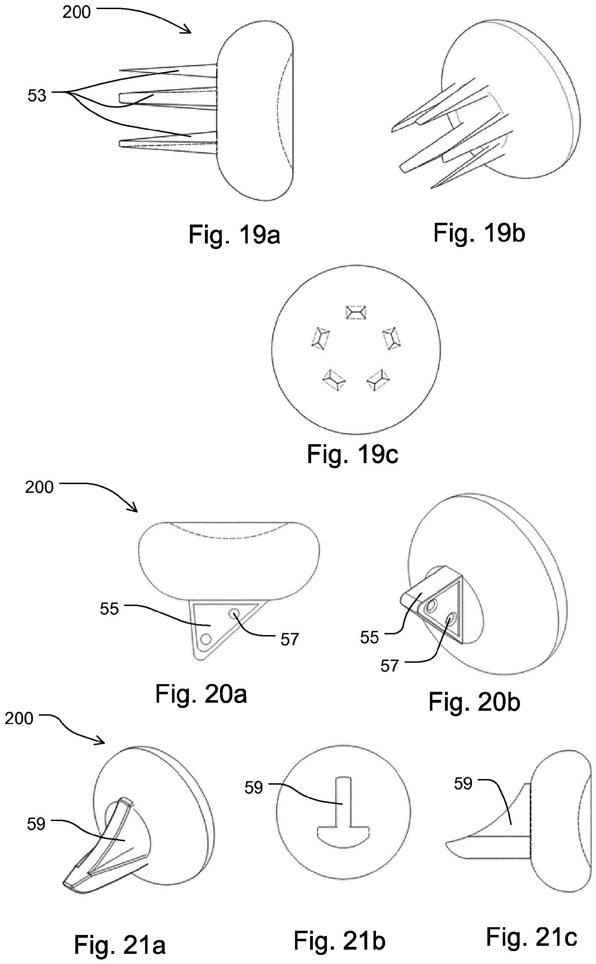

Der keilförmige Schaft 49 kann, ebenso wie die nachfolgend beschriebenen Verankerungsvorrichtungen (siehe Fig. 19, 20, 21) beschichtet sein. Eine Beschichtung kann eine sogenannte Osteointegration (Knocheneinwachsverhalten oder Knochenanwachsverhalten) verbessern oder zumindest positiv beeinflussen. Eine Beschichtung kann beispielsweise Reintitan aufweisen, das in einem sogenannten Plasma-Spray-Verfahren mit einer Schichtdicke von ca. 40 - 50 µm und einer Rauheit von ca. 7 µm aufgetragen wird. Alternativ kann eine Beschichtung Hydroxylapatit, das als sogenannter Knochenzement bezeichnet wird, aufweisen. Andere Beschichtungsmaterialien und Verfahren sind ebenso möglich.

Der keilförmige Schaft 49 in Fig. 18a und 18b weist eine abgerundete Spitze auf, die einer zusätzlichen oder weiteren Schädigung des Knochens bei einer Implantation entgegenwirken kann. Die seitlichen Einkerbungen 51 des keilförmigen Schafts 49 können eine Osteointegration begünstigen. Weiterhin können seitlichen Einkerbungen 51 die Stabilität des Radiuskopfimplantats 200, beispielsweise gegen eine Rotation um die Längsachse, erhöhen. Die seitlichen Einkerbungen 51 können als Hinterschneidungen bezeichnet werden.

In den **Fig. 19a, Fig. 19b** und **Fig. 19c** werden ein erfindungsgemäßes Radiuskopfimplantat 200 mit keilförmigen Dornen 53 als Verankerungsvorrichtung dargestellt.

Die keilförmigen Dornen 53 können als flexible Spikes bezeichnet werden, die sich beim Einbringen in den Knochen (oder Markraum) an die jeweilige Oberfläche anpassen. Die Dornen 53 können nach dem Einbringen nach innen gebogen werden, um dadurch eine Stabilität durch Spannung in den gegen den Knochen drückenden Dornen 53 zu erzielen. Optional können die Dornen 53 an der Spitze Widerhaken aufweisen, die sich in den Knochen "eingraben", wodurch eine zusätzliche Sicherung gegen ein Herausrutschen und/oder eine Rotation erreicht werden kann.

In den **Fig. 20a** und **Fig. 20b** werden ein weiteres erfindungsgemäßes Radiuskopfimplantat 200 mit einem kurzen Keil 55 als Verankerungsvorrichtung dargestellt.

Die Stabilität des Radiuskopfimplantats 200 kann überwiegend durch das Positionieren und Aufliegen des Kopfes (oberer Teil) des Radiuskopfimplantats 200 auf der Kortikalis des Radiushalses erzielt werden. Der kurze Keil 55 kann in den Markraum, oder wenn noch spongiöser Knochen vorhanden ist in diesen Bereich, eingebracht werden. Durch die geringe Höhe des Keils kann eine weite Aufspreizung des Gelenkspalts, des Knochens oder Markraums vermieden werden. Dadurch können beispielsweise Weichteile und/oder Bänder geschont und die natürliche Gelenkstabilität erhalten werden. Die Löcher 57 können das Einwachsen des Knochens in den Keil 55 verbessern und somit eine langfristige Stabilität des Implantats 200 ermöglichen.

In den **Fig. 21a, Fig. 21b** und **Fig. 21c** werden ein weiteres erfindungsgemäßes Radiuskopfimplantat 200 mit einem T-förmigen Keil 59 als Verankerungsvorrichtung dargestellt.

Die T-Form des Keils 59 ermöglicht eine insgesamt hohe Stabilität des Radiuskopfimplantats 200 und ermöglicht generell ein größeres Gestaltungsspektrum des Radiuskopfimplantats 200. Die laterale Seite des Keils 59 (in Fig. 21b und 21c der obere Bereich) ist nach innen abgerundet, womit eine weite Aufspreizung des Gelenkspalts, des Knochens oder des Markraums vermieden wird.

Die mediale Seite des Keils 59 (in Fig. 21b und 21c der untere Bereich) ist breit und abgerundet, damit eine möglichst große Kontaktfläche zum Knochen entstehen kann. Die Spitze des Keils 59 ist abgerundet, damit ein Einschneiden in den Knochen beim Einführen des Radiuskopfimplantats 200 vermieden werden kann und der Knochen nicht zusätzlich geschädigt wird. Der Keil 59 weist zum Kopf hin ein Plateau auf, so dass sich der Keil 59 beidseitig am Knochen abstützen kann. Der Keil 59 kann rein exemplarisch eine Höhe von ca. 15 mm aufweisen.

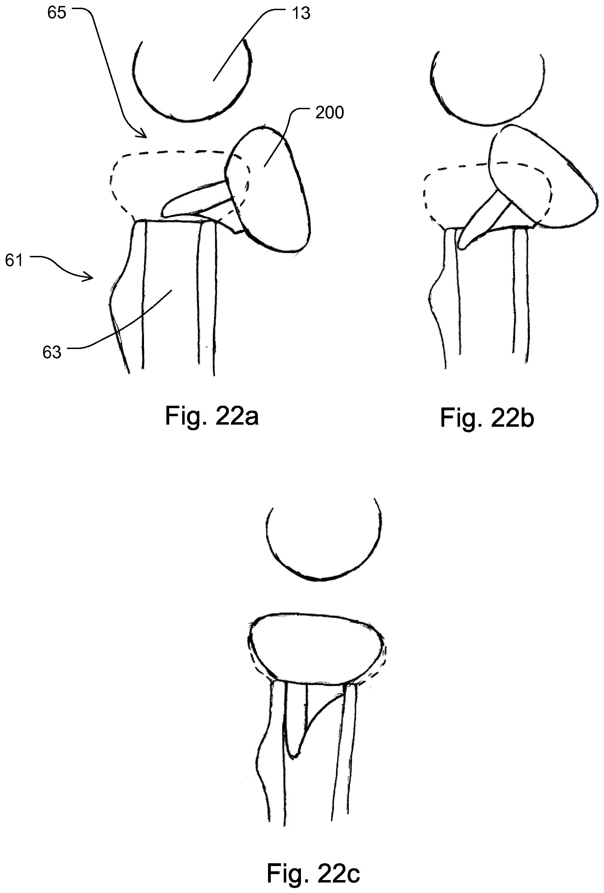

In den **Fig. 22a, Fig. 22b** und **Fig. 22c** werden schematisch die einzelnen Schritte zum Einbringen eines Radiuskopfimplantats 200 in den Markraum 63 des proximalen Radius 61 gezeigt. Gestrichelt dargestellt ist die Position des natürlichen, bereits entfernten, Radiuskopfes.

Der Gelenkspalt 65 zwischen dem Capitulum 13 und dem proximalen Radius 61 kann beispielsweise bereits durch abgetragenes Knochenmaterial am Capitulum 13 oder durch ein atraumatisches Spreizen, z. B. um bis zu 5 mm, aufgeweitet sein, damit das Radiuskopfimplantat 200, wie in den Fig. 22a bis 22c gezeigt, eingesetzt werden kann.

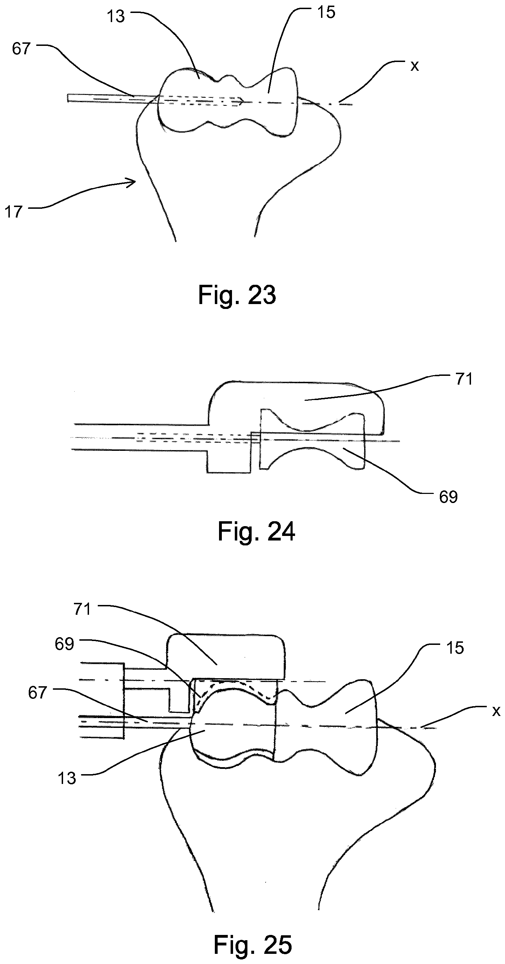

**Fig. 23** zeigt das Einführen eines K-Drahtes 67 in den distalen Humerus 17 zur Vorbereitung des Implantierens eines erfindungsgemäßen Capitulumimplantats 100. Der K-Draht 67, der rein exemplarisch einen Durchmesser von ca. 2 mm aufweist, wird von lateral (in Fig. 23 von links) über die Rotationsachse x zunächst in das Capitulum und anschließend in die Trochlea eingebracht. Die Rotationsachse x ist die natürliche Rotationsachse des dargestellten Gelenks.

Der K-Draht 67 kann als Bohrdraht 67 bezeichnet werden.

**Fig. 24** zeigt einen Fräskopf 69 mit einem Gewebeschutz 71 zum spanenden Abtragen von Knochenstrukturen. Die Form des Fräskopfes 69 (der Fräskopf 69 kann als Shaver bezeichnet werden) weist eine annähernd anatomische Form auf, um möglichst wenig Knochenmaterial an der Oberfläche abzutragen. Alternativ kann der Fräskopf 69 eine vereinfachte Form einer Tonne oder eine parabolische Form aufweisen. Der Gewebeschutz 71 bleibt während des Fräsvorgangs unbewegt. Der Fräskopf 69 rotiert um seine eigene Rotationsachse und trägt somit den Knochen ab.

**Fig. 25** zeigt den auf den K-Draht 67 aufgeschobenen Fräskopf 69 mit dem Gewebeschutz 71 beim Abtrag von Knochenstrukturen.

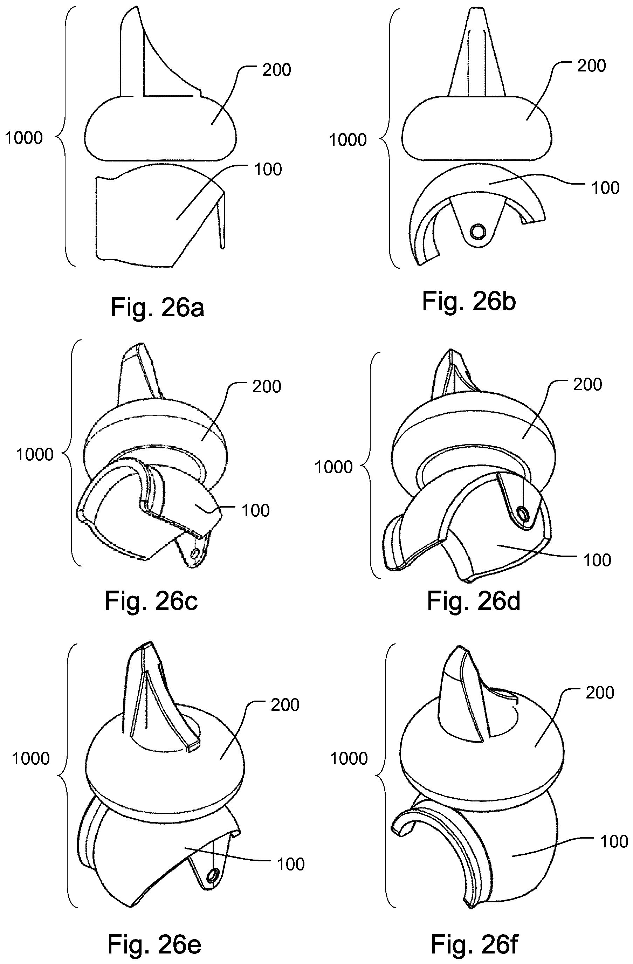

In den **Fig. 26** **a** - **f** werden in verschiedenen Ansichten der erfindungsgemäße partielle Ellenbogengelenkersatz 1000 mit einem Capitulumimplantat als schalenförmigem Segment 100 und einem Radiuskopfimplantat 200 dargestellt.

### Bezugszeichenliste

- 1000: partieller Ellenbogengelenkersatz Capitulumimplantat; Implantat (Teil eines partiellen Ellenbogengelenkersatzes);
- 100: schalenförmiges Segment
- 200: Radiuskopfimplantat (Teil eines partiellen Ellenbogengelenkersatzes)
- x: x-Richtung; Längsrichtung; Längsachse
- r: Radialrichtung; Richtung senkrecht zur Längsrichtung
- u: Umfangsrichtung
- X1: Länge des partieller Ellenbogengelenkersatzes/ Implantat Abstand des Implantats in x-Richtung vom medialen Ende bis zur
- X2: Längsposition mit dem maximalen Radius des Implantats Abstand des Implantats in x-Richtung von der Längsposition mit dem kleinsten
- X3: Radius zwischen Capitulum und Trochlea und der Längsposition mit dem maximalen Radius des Implantats
- R1: Maximaler Radius des Implantats senkrecht zur Längsrichtung Kleinster Radius des Implantats zwischen Capitulum und Trochlea senkrecht
- R2: zur Längsrichtung Äußerer Radius an der medialen Stirnseite des Implantats senkrecht zur
- R3: Längsrichtung
- R4: Radius der Capitulum-Oberfläche des Implantats Radius der Implantat-Oberfläche in Längsrichtung zwischen Capitulum und
- R5: Trochlea
- α: Winkel zur Radialrichtung
- β1, β2: Umfangswinkel
- 1: äußere Oberflächenform, Außenkontur
- 3: innere Oberflächenform, Innenkontur
- 5: laterales Ende; lateraler Endbereich; laterale Stirnseite; lateraler Abschnitt
- 7: mediales Ende; medialer Endbereich; mediale Stirnseite, medialer Abschnitt
- 9: Wendepunkt
- 11: Ellenbogengelenk
- 13: Capitulum
- 15: Trochlea
- 17: Humerus; distaler Humerus
- 19: Längsachse des Humerus; Schaftachse des Humerus
- 21: zylindrische Innenkontur
- 23: zylindrischer Absatz
- 25: Einkerbung in Umfangsrichtung u
- 27: Einkerbung in Längsrichtung x
- 29: kegelförmiger Schaft (als Verankerungsvorrichtung)
- 31: trapezförmiger Schaft (als Verankerungsvorrichtung)
- 33: Keil (als Verankerungsvorrichtung)
- 35: Widerhaken
- 37: Kante (in Umfangsrichtung)
- 39, 39': Steg zur Schraubenfixierung
- 41, 41': Öffnung, Bohrung
- 43: Scharnier
- 45: Tellertiefe
- 47: Außenwand des Radiuskopfimplantats
- 49: keilförmiger Schaft
- 51: seitliche Einkerbung
- 53: keilförmiger Dorn
- 55: kurzer Keil
- 57: Loch (im kurzen Keil)
- 59: T-förmiger Keil
- 61: proximaler Radius
- 63: Markraum
- 65: Gelenkspalt
- 67: K-Draht; Kirschner-Draht
- 69: Fräskopf
- 71: Gewebeschutz
- M1, M2, M3: Schnittebenen

## Patentansprüche

1. Partieller Ellenbogengelenkersatz (1000), umfassend:
- ein schalenförmiges Segment (100) entlang einer Längsrichtung (x) mit einer konkaven Innenkontur (3) zum Anordnen auf oder an Knochenstrukturen eines Patienten, wobei das Segment (100) einen lateralen Abschnitt (5) und einen medialen Abschnitt (7) aufweist;
- wobei eine Außenkontur (1) des Segments (100) in Längsrichtung (x) wenigstens einen Wendepunkt (9) aufweist; und
- wobei die Innenkontur (3) des Segments (100) in Längsrichtung (x) wenigstens einen Wendepunkt (9) aufweist.

2. Partieller Ellenbogengelenkersatz (1000) gemäß Anspruch 1, wobei das schalenförmige Segment (100) eine raue Innenkontur-Oberfläche (3) aufweist.

3. Partieller Ellenbogengelenkersatz (1000) gemäß einem der vorangegangenen Ansprüche, wobei die Innenkontur (3) des Segments (100) in Längsrichtung (x) analog der Außenkontur (1) des Segments (100) ausgebildet ist.

4. Partieller Ellenbogengelenkersatz (1000) gemäß einem der Ansprüche 1 bis 3, wobei die Innenkontur (3) des lateralen Abschnitts des Segments (100) eine konkave, nicht-zylindrische Kontur aufweist, und wobei die Innenkontur (3) des medialen Abschnitts des Segments (100) eine zylindrische Kontur in Längsrichtung (x) aufweist.

5. Partieller Ellenbogengelenkersatz (1000) gemäß einem der Ansprüche 1 bis 3, wobei die Innenkontur (3) des lateralen Abschnitts des Segments (100) eine konkave, nicht-zylindrische Kontur aufweist, und wobei die Innenkontur (3) des medialen Abschnitts des Segments (100) eine nach radial innen gewölbte, konvexe Kontur in Längsrichtung (x) aufweist.

6. Partieller Ellenbogengelenkersatz (1000) gemäß einem der vorangegangenen Ansprüche, wobei das schalenförmige Segment (100) an seinem lateralen, stirnseitigen Endabschnitt eine Fixiereinrichtung zum Fixieren des schalenförmigen Segments (100) am Knochen aufweist.

7. Partieller Ellenbogengelenkersatz (1000) gemäß dem vorangegangenen Anspruch, wobei die Fixiereinrichtung eine Schraube umfasst.

8. Partieller Ellenbogengelenkersatz (1000) gemäß Anspruch 6 oder 7, welcher mittels der Fixiereinrichtung parallel zur Längsrichtung (x) im Knochen fixierbar ist.

9. Partieller Ellenbogengelenkersatz (1000) gemäß einem der vorangegangenen Ansprüche, wobei das Segment (100) aus einem metallischen Werkstoff und/oder einem keramischen Werkstoff und/oder einem Verbundwerkstoff und/oder Polymerwerkstoff hergestellt ist oder einen solchen jeweils aufweist.

10. Partieller Ellenbogengelenkersatz (1000) gemäß einem der vorangegangenen Ansprüche, wobei das Segment (100) an seiner Innenseite (100) wenigstens eine Verankerungsvorrichtung zum Verankern des Segments (100) in einer Knochenstruktur aufweist.

11. Partieller Ellenbogengelenkersatz (1000) gemäß einem der vorangegangenen Ansprüche, wobei die Längsachse (x) parallel zu einer Drehachse des Ellenbogengelenks verläuft, im implantierten Zustand des Ellenbogengelenkersatzes (1000) eine Drehachse des Ellenbogengelenks, und/oder eine Epicondylus-Achse des Ellenbogengelenks ist.

12. Partieller Ellenbogengelenkersatz (1000) gemäß einem der vorangegangenen Ansprüche, wobei die Innenseite (3) des Segments (100) derart geformt ist, dass sie zu einer Fläche einer proximalen Ulna des Ellenbogengelenks konform ist.

13. Partieller Ellenbogengelenkersatz (1000) gemäß einem der vorangegangenen Ansprüche, wobei das Segment (100) lateral mit einer Eintritts- oder Öffnungsebene oder Wandung endet, welche in einer Ebene liegt, die unter einem Winkel (a) zur Längsachse (x) steht.

14. Partieller Ellenbogengelenkersatz (1000) gemäß dem vorangegangenen Anspruch, wobei der Winkel (α) in einem Bereich von 25° bis 45°, bevorzugt zwischen 30° und 40°, liegt und insbesondere bevorzugt 35° beträgt.

15. Partieller Ellenbogengelenkersatz (1000) gemäß einem der vorangegangenen Ansprüche, weiterhin umfassend:
- ein Radiuskopfimplantat (200).

## Claims

1. A partial elbow joint replacement (1000), comprising:
- a shell-like segment (100) along a longitudinal direction (X) having a concave inner contour (3) for arrangement on or at bone structures of a patient, the segment (100) comprising a lateral section (5) and a medial section (7);
- wherein an outer contour (1) of the segment (100) comprises at least one inflection point (9) in the longitudinal direction (X); and
- wherein the inner contour (3) of the segment (100) comprises at least one inflection point (9) in the longitudinal direction (X).

2. The partial elbow joint replacement (1000) according claim 1, wherein the shell-like segment (100) comprises a rough inner contour surface (3).

3. The partial elbow joint replacement (1000) according to anyone of the preceding claims, wherein the inner contour (3) of the segment (100) is designed in the longitudinal direction (X) analogous to the outer contour (1) of the segment (100).

4. The partial elbow joint replacement (1000) according to anyone of claims 1 to 3, wherein the inner contour (3) of the lateral section of the segment (100) comprises a concave, non-cylindrical contour, and wherein the inner contour (3) of the medial section of the segment (100) comprises a cylindrical contour in the longitudinal direction (X).

5. The partial elbow joint replacement (1000) according to anyone of claims 1 to 3, wherein the inner contour (3) of the lateral section of the segment (100) comprises a concave, non-cylindrical contour, and wherein the inner contour (3) of the medial section of the segment (100) comprises a convex contour in the longitudinal direction (X), which is radially bent to the inside.

6. The partial elbow joint replacement (1000) according to anyone of the preceding claims, wherein the shell-like segment (100) comprises a fixing device at its lateral, front end portion for fixing the shell-like segment (100) to the bone.

7. The partial elbow joint replacement (1000) according to the preceding claim, wherein the fixing device comprises a screw.

8. The partial elbow joint replacement (1000) according to claim 6 or 7, which can be fixed in the bone parallel to the longitudinal direction (X) by means of the fixing device.

9. The partial elbow joint replacement (1000) according to anyone of the preceding claims, wherein the segment (100) is made of a metallic material and/or a ceramic material and/or a composite material and/or a polymer material, or respectively comprises such a material.

10. The partial elbow joint replacement (1000) according to anyone of the preceding claims, wherein the segment (100) comprises at least one anchoring apparatus at its inner side for anchoring the segment (100) in a bone structure.

11. The partial elbow joint replacement (1000) according to anyone of the preceding claims, wherein the longitudinal axis (X) extends parallel to an axis of rotation of the elbow joint, and, in a state in which the elbow joint replacement (1000) is implanted, is an axis of rotation of the elbow joint, and/or an epicondyle axis of the elbow joint.

12. The partial elbow joint replacement (1000) according to anyone of the preceding claims, wherein the inner side of the segment (100) is shaped in such a way that it conforms to a surface of a proximal ulna of the elbow joint.

13. The partial elbow joint replacement (1000) according to anyone of the preceding claims, wherein the segment (100) ends laterally with an inlet or opening plane or wall which lies in a plane being under an angle (a) relative to the longitudinal axis (X).

14. The partial elbow joint replacement (1000) according to the preceding claim, wherein the angle (α) has a value within a range of 25° to 45°, preferably between 30° and 40°, and more particularly is preferably 35°.

15. The partial elbow joint replacement (1000) according to anyone of the preceding claims, further comprising:
- a radius head implant (200).

## Revendications

1. Une prothèse articulaire partielle du coude (1000), comprenant:
- un segment en forme de coque (100) le long d'une direction longitudinale (X) ayant un contour intérieur concave (3) pour un agencement sur ou contre des structures osseuses d'un patient, le segment (100) comprenant une section latérale (5) ainsi qu'une section médiane (7);
- où un contour extérieur (1) du segment (100) comprend au moins un point d'inflexion (9) dans la direction longitudinale (X); et
- où le contour intérieur (3) du segment (100) comprend au moins un point d'inflexion (9) dans la direction longitudinale (X).

2. La prothèse articulaire partielle du coude (1000) selon la première revendication, où le segment en forme de coque (100) comprend une surface de contour intérieur rugueuse (3).

3. La prothèse articulaire partielle du coude (1000) selon l'une quelconque des revendications précédentes, où le contour intérieur (3) du segment (100) est conçu dans la direction longitudinale (X) de manière analogue au contour extérieur (1) du segment (100).

4. La prothèse articulaire partielle du coude (1000) selon l'une quelconque des revendications 1 à 3, où le contour intérieur (3) de la section latérale du segment (100) comprend un contour concave, non cylindrique, et où le contour intérieur (3) de la section médiane du segment (100) comprend un contour cylindrique dans la direction longitudinale (X).

5. La prothèse articulaire partielle du coude (1000) selon l'une quelconque des revendications 1 à 3, où le contour intérieur (3) de la section latérale du segment (100) comprend un contour concave, non cylindrique, et où le contour intérieur (3) de la section médiane du segment (100) comprend un contour convexe incurvé radialement vers l'intérieur dans la direction longitudinale (X).

6. La prothèse articulaire partielle du coude (1000) selon l'une quelconque des revendications précédentes, où le segment en forme de coque (100) comprend un dispositif de fixation à son extrémité latérale et frontale pour fixer le segment en forme de coque (100) à l'os.

7. La prothèse articulaire partielle du coude (1000) selon la revendication précédente, où le dispositif de fixation comprend une vis.

8. La prothèse articulaire partielle du coude (1000) selon la revendication 6 ou 7, qui peut être fixée dans l'os parallèlement à la direction longitudinale (X) au moyen du dispositif de fixation.

9. La prothèse articulaire partielle du coude (1000) selon l'une quelconque des revendications précédentes, où le segment (100) est constitué d'un matériau métallique et/ou d'un matériau céramique et/ou d'un matériau composite et/ou d'un matériau polymère, ou comprend respectivement un tel matériau.

10. La prothèse articulaire partielle du coude (1000) selon l'une quelconque des revendications précédentes, où le segment comprend au moins un appareil d'ancrage sur sa face interne pour ancrer le segment (100) dans une structure osseuse.

11. La prothèse articulaire partielle du coude (1000) selon l'une quelconque des revendications précédentes, où l'axe longitudinal (X) s'étend parallèlement à un axe de rotation de l'articulation du coude, et, lorsque la prothèse articulaire du coude (1000) est implantée, est un axe de rotation de l'articulation du coude, et/ou un axe épicondyle de l'articulation du coude.

12. La prothèse articulaire partielle du coude (1000) selon l'une quelconque des revendications précédentes, où la face interne du segment (100) est formée de telle manière qu'elle se conforme à une surface d'un ulna proximal de l'articulation du coude.

13. La prothèse articulaire partielle du coude (1000) selon l'une quelconque des revendications précédentes, où le segment (100) se termine latéralement par un plan ou une paroi d'entrée ou d'accès qui se situe dans un plan formant un angle (a) avec l'axe longitudinal (X).

14. La prothèse articulaire partielle du coude (1000) selon la revendication précédente, où l'angle (a) se situe dans une plage de 25° à 45°, de préférence entre 30° et 40°, et vaut notamment de préférence 35°.

15. La prothèse articulaire partielle du coude (1000) selon l'une quelconque des revendications précédentes, comprenant en outre:
- un implant de tête de rayon (200).
